# EUROPÄISCHE PATENTSCHRIFT

(11) **EP 1 435 914 B1**
(45) Veröffentlichungstag und Bekanntmachung des Hinweises auf die Patenterteilung: **12.06.2013**
(21) Anmeldenummer: 02779459.3
(22) Anmeldetag: 02.10.2002
(51) Int. Cl.: A61K 9/14, A61K 9/19, A61K 38/16

(54) **STABILE GALENISCHE GEFRIERGETROCKNETE ARZNEIMITTELZUBEREITUNG VON REKOMBINANTEN CARBOHYDRATBINDENDEN POLYPEPTIDEN**
STABLE GALENIC FREEZE-DRIED PHARMACEUTICAL PREPARATION OF RECOMBINED CARBOHYDRATE-BINDING POLYPEPTIDES
PREPARATION PHARMACEUTIQUE LYOPHILISEE GALENIQUE STABLE DE POLYPEPTIDES DE RECOMBINAISON LIANT LES HYDROCARBURES

(30) Priorität: 05.10.2001 DE 10149030
(43) Veröffentlichungstag der Anmeldung: 14.07.2004
(73) Patentinhaber: Viscum AG, 51429 Bergisch-Gladbach (DE)
(72) Erfinder: GLOGER, Olivier, 52066 Aachen (DE); MÜLLER, Bernd, W., 24220 Flintbeck (DE); WITTHOHN, Klaus, 51491 Overath (DE)
(74) Vertreter: Simandi, Claus
(86) Internationale Anmeldenummer: PCT/EP2002/011093
(87) Internationale Veröffentlichungsnummer: WO 2003/030866

(56) Entgegenhaltungen:
- EP-A- 0 430 200
- EP-A- 0 751 221
- DE-A- 4 221 836
- DE-A- 19 716 154
- US-A- 5 710 257
- US-B1- 6 238 664

## Beschreibung

Die Erfindung betrifft ein Verfahren zur Herstellung eines Arzneimittels enthaltend ein Polypeptid umfassend mindestens ein rekombinantes carbohydratbindendes Polypeptid oder funktionelles Fragment oder Derivat dieses carbohydratbindenden Polypeptids in einer lagerungsstabilen Form. Das genannte Polypeptid umfaßt Polypeptide oder funktionelle Derivate davon, welche mit zytotoxisch wirkenden Peptiden zu Fusionsproteinen fusioniert sind, oder welche mit einem weiteren Polypeptid verbunden sind, welches eine zytotoxische Aktivität besitzt. Darüber hinaus beschreibt die Erfindung die Weiterformulierung der offenbarten Arzneimittel zu Arzneimittel verschiedener Darreichungsformen.

Die medizinische Forschung hat in den letzten Jahren ein breites Spektrum von Erkrankungen aufgedeckt, die durch rekombinante Proteine behandelt werden können. Beispiele für Proteine humanen Ursprungs sind Insulin, EPO und G-CSF, deren Darreichungsformen und Applikationsarten in verschiedenen europäischen Patentschriften beschrieben wurden. EP 0 430 200 B1 beschreibt die Anwendung von Humanproteinen zur subkutanen und intramuskulären Anwendung. Arzneimittel mit stabilisierten humanen Proteinen, die unter anderem Harnstoff oder verschiedene Aminosäuren enthalten, sind aus EP 0 306 824 B1 bekannt. In diesem Patent werden als Beispiele EPO und G-CSF erwähnt. EP 0 607 156 B1 beschreibt die Herstellung von konservierten Arzneimitteln mit Humanproteinen für Infusions- oder Injektionszwecke.

Allgemein bezieht sich der Begriff "rekombinant" auf solche Proteine, die mit Hilfe der rekombinanten DNA-Technologie hergestellt werden. Diese Verfahren umfassen die Klonierung des Gens, welches für das jeweilige Protein kodiert, die Insertion der entsprechenden cDNA oder genomischen DNA in ein geeignetes Vektorsystem und die Transformation/Transfektion dieser Vektoren in geeignete Wirtsorganismen (Bakterien oder eukaryotische Zellen). Wird das klonierte Gen in dem Wirtsorganismus exprimiert, so kann das entsprechende Protein aus Kulturüberstand (wenn das exprimierte Protein sezerniert wird) oder aus einem Homogenisat des Wirtsorganismus (wenn das entsprechende Protein intrazellulär exprimiert wird) gewonnen werden. Verfahren zur Herstellung rekombinanter Proteine sind sowohl für tierische als auch für pflanzliche Proteine beschrieben. Ein Beispiel für die genaue Vorgehensweise für die Herstellung eines dimeren pflanzlichen Proteins wird in EP 0 751 221 B1 beschrieben. Dieses Patent beschreibt unter anderem die erstmalige erfolgreiche Klonierung der die ML-Untereinheiten kodierenden Gene. Darüber hinaus wird in diesem Patent auch die Verwendung dieser dimeren, rekombinant hergestellten, pflanzlichen Proteine zur Herstellung von Arzneimitteln beschrieben.

Die Verwendung von Mistelextrakten (Extrakte von Viscum album) als Heilmittel sind schon seit Jahrhunderten bekannt. Als wirksamer Bestandteile dieser Extrakte wurden hierbei als Lektine bezeichnete Inhaltsstoffe identifiziert. Bei diesen Lektinen handelt es sich um Eiweißstoffe, die sehr spezifische Kohlenhydratstrukturen auch in lipid- oder proteingebundener Form erkennen und an diese binden. Mistellektin, das als Ribosomen inaktivierendes Protein der Klasse II charakterisiert wurde, wirkt pharmakologisch nur durch das Zusammenspiel seiner beiden Untereinheiten. Die B-Kette des Mistellektins, die Sequenzmotive mit spezifischen kohlenhydratbindenden Eigenschaften besitzt, ist hierbei für den Transport des Proteins in die Zielzelle verantwortlich. In der Zielzelle blockiert dann die A-Untereinheit durch ihre enzymatische rRNA-N-Glycosidase-Aktivität den ribosomalen Stoffwechsel der Zelle und löst auf diese Weise einen programmierten Zelltod (Apoptose) in dieser aus.

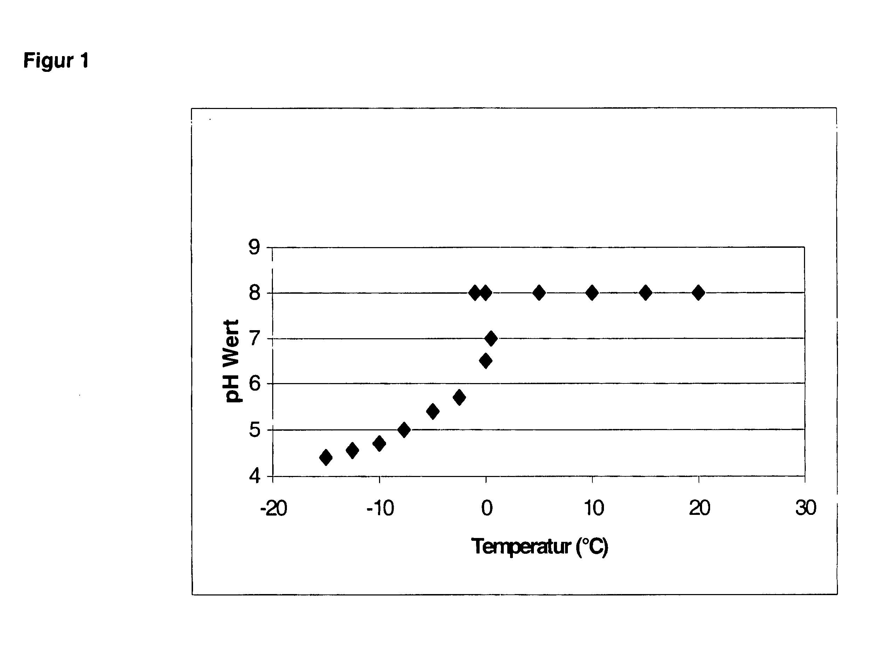

Die bisher aus dem Stand der Technik bekannten Arzneimittelpräparate enthalten im allgemeinen Humanproteine, humanisierte Proteine, Extrakte enthaltend pflanzliche Proteine oder aus Pflanzen isolierte Proteine. Entscheidend für die Wirksamkeit von Proteine enthaltenden Präparaten ist der Erhalt der biologischen Aktivität dieser Proteine. Für rViscumin ist dies zum Beispiel die dimere Struktur und der Erhalt der den Einzelketten zuzuordnenden Aktivitäten und der spezifischen pharmakologischen Wirkungsweise dieser Moleküle. Der Erhalt dieser biologischen Aktivitäten ist stark abhängig vom pH-Wert der die Proteine enthaltenden Lösung (siehe Figur 1). Darüber hinaus beeinflussen die Lagerungsbedingungen der jeweiligen Zubereitung die Stabilität eines Arzneistoffs/eines Arzneimittels.

In dem europäischen Patent EP 0 602 686 B1 wurde die Wirkungsweise der Mistelpflanze und der daraus gewonnenen Extrakte zur Behandlung von Krankheiten beschrieben. Mistelextrakte werden, wie in dieser Beschreibung ausgeführt, seit Jahrhunderten therapeutisch genutzt. Seit Anfang dieses Jahrhunderts werden Mistelpräparate zur Krebstherapie mit unterschiedlichem Erfolg angewandt (Bocci, 1993; Gabius et al., 1994; Gabius & Gabius, 1994; Ganguly & Das, 1994). Hajto et al. (1989, 1990) konnte zeigen, daß die therapeutischen Effekte insbesondere durch sogenannte Mistellektine (Viscumine, Viscum album Agglutinine, VAA) vermittelt werden. Es wird neben einer zytotoxischen Wirkung heute insbesondere eine (unspezifische) Immunstimulation diskutiert, deren positive Effekte zur begleitenden Therapie und zur Nachsorge von Tumorpatienten ausgenutzt werden. Eine Steigerung der Lebensqualität bei solchen Patienten wird möglicherweise durch die Ausschüttung körpereigener Endorphine vermittelt (Heiny und Beuth, 1994).

Zahlreiche Untersuchungen in vitro (Hajto et al., 1990; Männel et al., 1991; Beuth et al., 1993a) und in vivo (Hajto, 1986; Hajto et al., 1989, Beuth et al., 1991; Beuth et al., 1992), sowie klinische Studien (Beuth et al., 1992) belegen die durch Mistellektin vermittelte erhöhte Freisetzung von inflammmatorischen Zytokinen (TNF-α, IL-1, IL-6) sowie eine Aktivierung von zellulären Komponenten des Immunsystems (TH-Zellen, NK-Zellen).

Als aktives Prinzip der Mistelextrakte wird heute ein 60kDa Mistellektin-Protein angesehen, das auf biochemischen Weg aus Extrakten gewonnen werden kann (Franz et al., 1977; Gabius et al., 1992). Das ML-Protein besteht aus zwei kovalent S-S verbrückten Untereinheiten, dessen A-Kette für eine enzymatische Inaktivierung von Ribosomen (Endo et al., 1988) und dessen B-Kette für die Carbohydratbindung verantwortlich ist. Die biologische Aktivität wird korreliert mit dem Erhalt der Lektinaktivität der B-Kette (Hajto et al., 1990).

Die Verwendung einer Arzneiform bzw. einer Arzneizubereitung mit rViscumin als aktiver Komponente stellt eine interessante und vorteilhafte Alternative zur pflanzlichen Zubereitung dar, da sich nun die Möglichkeit ergibt, eine chemisch klassifizierte Substanz als Medikament einzusetzen. Gerade im Hinblick auf die hohe Toxizität des Mistellektins ist durch die Verwendung von rekombinant hergestellten Proteinen eine gute Verträglichkeit durch eine exakte Dosierung möglich. Von besonderem Vorteil ist hierbei eine Arzneiform bzw. eine Arzneizubereitung, die über einen langen Zeitraum, d.h. über mehrere Monate und vorzugsweise mindestens ein Jahr, lagerungsstabil ist. Die Lagerung der Arzneiform bzw. Arzneizubereitung in dieser lagerungsstabilen Form sollte darüber hinaus einfach und ohne großen technischen Aufwand möglich sein. Des weiteren sollte die Arzneiform bzw. Arzneizubereitung, wenn dessen lagerungsstabile Form nicht der Darreichungsform entspricht, einfach, zu einer entsprechenden Darreichungsform weiterformulierbar sein. Mit wässrigen Rezepturen nach dem Stand der Technik sind Lagerungszeiten von unter 10 Wochen (2,5 Monate) bei den Lagerungsbedingungen 2 - 8 °C (Kühlschrank) zu realisieren.

Das der vorliegenden Erfindung zugrundeliegende technische Problem war somit die Bereitstellung eines Verfahrens zur Herstellung eines Arzneimittels oder Arzneimittelzubereitung in einer langzeit-lagerungsstabilen Form, die eine einfache Handhabung sowohl während der Lagerung, als auch bei der Verabreichung und gegebenenfalls bei deren Vorbereitung gewährleistet. Das erfindungsgemäße Arzneimittel soll hierbei mindestens ein rekombinantes carbohydratbindendes Polypeptid oder ein funktionelles Fragment oder Derivat dieses Polypeptids umfassen, darüber hinaus gegebenenfalls enthaltend einen pharmakologisch verträglichen Träger.

Dieses technische Problem wird durch die in den Ansprüchen charakterisierten Ausführungsformen gelöst.

Folglich betrifft die vorliegende Erfindung ein Verfahren zur Herstellung eines Arzneimittels, enthaltend ein Polypeptid, umfassend mindestens ein rekombinantes carbohydratbindendes Polypeptid einer B-Kette eines Ribosomen-inaktivierenden Proteins oder funktionelles Fragment oder Derivat dieses Polypeptids, in einer langzeit-lagerungsstabilen Form, darüber hinaus gegebenenfalls enthaltend einen pharmakologisch verträglichen Träger, umfassend den Schritt des Abkühlens, Einfrierens, Sprühtrocknens oder Gefriertrocknens unter Erhalt der pharmakologischen Eigenschaften des Polypeptids in der Lösung, wobei die Lösung dadurch gekennzeichnet ist, dass der ph-Wert der Lösung größer als ph 6,0 ist und ein im Lösungsmittel enthaltenes Puffersystem die Aufrechterhaltung dieses ph-Werts gewährleistet.

Rekombinante Polypeptide und Proteine können ausgehend von den entsprechenden klonierten Genen unter Anwendung von konventionellen molekularbiologischen Methoden dargestellt werden. Diese werden unter anderem in dem Lehrbuch "Gentechnologie" (Old und Primrose, 1992) oder in den Laborhandbüchern "Methods for General and Molecular Bacteriology" (Gerhardt et al., Chapter 18) oder "Molecular Cloning: A Laboratory Manual" (Sambrook et al. 1993) beschrieben.

Erfindungsgemäß ist ein "carbohydratbindendes Polypeptid" ein Polypeptid, welches die Eigenschaft besitzt, spezifisch an bestimmte Carbohydrate zu binden. Beispiele für solche Carbohydrate sind Galaktose, N-Acetyl-Galaktosamin, modifizierte Galaktose, Neuraminsäuren, niedermolekulare Saccharide und Oligosaccharide mit endständiger Galaktose und/oder endständiger Galaktosamineinheiten bzw. modifizierten Galaktoseeinheiten oder endständiger Neuraminsäureeinheiten, und Peptide und Lipide mit entsprechender Carbohydratfunktion. Erfindungsgemäß "funktionelle Fragmente oder Derivate dieses Polypeptids" sind dadurch charakterisiert, dass diese ebenso eine Spezifität für die Bindung an die oben genannten Carbohydrate besitzen.

Die Verwendung von erfindungsgemäßen Polypeptiden, wie z. B. rViscumin und andere pflanzliche, dimere Polypeptide der Klasse II der Ribosomen-inaktivierenden-Proteine (RIP II) zur Herstellung von hochwirksamen Arzneimitteln ist unter anderem in EP 0 751 221 B1 beschrieben. Diese Arzneimittel werden bevorzugt, jedoch spätestens nach einem Jahr nach der Herstellung verabreicht.

Ein Arzneimittel oder eine Arzneimittelzubereitung gilt im Sinne der Erfindung als lagerungsstabil, wenn dies über einen langen Zeitraum, d. h. mehrere Monate und zwar mindestens über sechs Monate, gelagert werden kann, ohne dass ein signifikante Veränderung der spezifischen Eigenschaften der Arzneimittelzubereitung und des Polypeptids und der damit verbundenen Wirksamkeit dieses Arzneimittels oder der Zubereitung beobachtet wird. Bevorzugt ist in diesem Zusammenhang eine lagerungsstabile Form von erfindungsgemäßen Arzneimitteln oder Arzneimittelzubereitungen, die über einen Zeitraum von 1, 2, 3, 4 oder 5 Jahren gelagert werden, bevorzugt unter marktüblichen und durch die Distributoren und Anwender einzuhaltende Lagerungsbedingungen (2-8°C und/oder Umgebungstemperatur unter 25°C) gelagert werden können, ohne daß ein signifikante Veränderung der spezifischen Eigenschaften der Arzneimittelzubereitung und des Polypeptids und der damit verbundenen Wirksamkeit dieses Arzneimittels oder der Zubereitung eintritt. Daher betrifft die Erfindung besonders gut handhabbare Lagerungs- und Transportformen der hierin beschriebenen Polypeptide.

Die erfindungsgemäße Arzneimittelformulierung erfolgt gegebenenfalls in Kombination mit einem "pharmakologisch verträglichen Träger" und/oder Verdünnungsmittel. Beispiele für besonders geeignete pharmakologisch verträgliche Träger sind dem Fachmann bekannt und umfassen gepufferte Kochsalzlösungen, Wasser, Emulsionen wie z.B. Öl/Wasser-Emulsionen, verschiedene Arten von Detergenzien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden. Die Verabreichung kann oral oder parenteral erfolgen, z.B. intravenös, intraperitoneal, subcutan, intramuskulär, lokal, intranasal, intrabronchial oder intradermal, oder über einen Katheter an einer Stelle in einer Arterie. Die Art der Dosierung wird vom behandelnden Arzt entsprechend den klinischen Faktoren bestimmt. Es ist dem Fachmann bekannt, daß die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden. Eine typische Dosis kann z.B. in einem Bereich zwischen 0,001 und 1000 µg liegen, wobei Dosen unterhalb oder oberhalb dieses beispielhaften Bereiches, vor allem unter Berücksichtigung der oben erwähnten Faktoren, vorstellbar sind. Im allgemeinen sollte sich bei regelmäßiger Verabreichung der erfindungsgemäßen Zusammensetzung die Dosis in einem Bereich zwischen 10 ng- und 10 mg-Einheiten pro Tag bzw. pro Applikationsintervall befinden. Wird die Zusammensetzung intravenös verabreicht sollte sich die Dosis in einem Bereich zwischen 1 ng- und 0,1 mg-Einheiten pro Kilogramm Körpergewicht pro Minute befinden.

Die Zusammensetzung der Erfindung kann lokal oder systemisch verabreicht werden. Präparate für eine parenterale Verabreichung umfassen sterile wäßrige oder nicht-wäßrige Lösungen, Suspensionen und Emulsionen. Beispiele für nicht-wäßrige Lösungsmittel sind Propylenglykol, Polyethylenglykol, pflanzliche Öle wie z.B. Olivenöl, und organische Esterverbindungen wie z.B. Ethyloleat, die für Injektionen geeignet sind. Wäßrige Träger umfassen Wasser, alkoholisch-wäßrige Lösungen, Emulsionen, Suspensionen, Salzlösungen und gepufferte Medien. Parenterale Träger umfassen Natriumchlorid-Lösungen, Ringer-Dextrose, Dextrose und Natriumchlorid, Ringer-Laktat und gebundene Öle. Intravenöse Träger umfassen z.B. Flüssigkeits-, Nährstoff- und Elektrolyt-Ergänzungsmittel (wie z.B. solche, die auf Ringer-Dextrose basieren). Die erfindungsgemäße Zusammensetzung kann außerdem Konservierungsmittel und andere Zusätze umfassen, wie z.B. antimikrobielle Verbindungen, Antioxidantien, Komplexbildner und inerte Gase. Des weiteren können, abhängig von der beabsichtigten Verwendung, Verbindungen wie z.B. Interleukine, Wachstumsfaktoren, Differenzierungsfaktoren, Interferone, chemotaktische Proteine oder ein unspezifisches immunmodulatorisches Agens enthalten sein.

Die verwendeten Puffersubstanzen sind geeignet, während der Phase des Abkühlens, Einfrierens, Sprühtrocknens oder Gefriertrocknens den eingestellten pH-Wert innerhalb der beschriebenen Bereiche aufrecht zu erhalten. Die Puffersubstanzen werden bevorzugt so gewählt, daß bei einer geringen Pufferkapazität eine Veränderung des eingestellten pH-Wertes der Lösung während des Gefriervorgangs zu niedrigeren Werten nicht möglich ist. Durch die Aufrechterhaltung eines hohen pH-Bereichs während des Gefriertrocknungsvorgangs wird die Stabilität des Polypeptids gewährleistet. Eine geringe Pufferkapazität ist des weiteren für eine fertig applizierbare Injektionslösung bevorzugt. In Beispiel 1 ist ein Verfahren zur Überprüfung des pH-Werts während der Abkühlung, bzw. des Gefrierens von Arzneimittelzubereitungen beschrieben. Mit Hilfe dieses oder ähnlicher Verfahren lassen sich Puffersubstanzen bestimmen, die für das erfindungsgemäße Verfahren geeignet sind.

Im Stand der Technik ist eine Vielzahl von Arzneimitteln beschrieben, die in gepufferten Lösungen niedermolekulare, oligomere Verbindungen, (incl. Peptide) und hoch molekulare Verbindungen (incl. Polypeptide) enthalten. Ebenfalls sind für eine Vielzahl von solchen Arzneimitteln, die entsprechende Verbindungen enthalten, welche über einen weiten pH-Bereich stabil sind, Verfahren zur Verbesserung der Lagerungseigenschaften beschrieben und dem Fachmann bekannte. Beispiele hierfür sind Verfahren, die das Einfrieren, Sprühtrocknen oder Gefriertrocknen der Arzneimittel umfassen. Aufgrund dieser pH-Wert unabhängigen Stabilität war bisher keine spezifische Kontrolle des pH-Werts während des Gefrier- oder Sprühtrocknungsvorgangs als notwendig beschrieben worden. Ebenso sind übliche Gefriertrocknungsanlagen zur Herstellung von Arzneimitteln und Arzneimittelzubereitungen nicht mit Vorrichtungen zur pH-Wert-Kontrolle ausgestattet.

Bei der Anwendung solcher bekannten Verfahren wurde überraschenderweise festgestellt, daß die Lektineigenschaften von rViscumin und anderer pflanzlicher, dimerer Polypeptide der Klasse II der Ribosomen-inaktivierenden-Proteine (RIP II) unter bestimmten Umständen sensitiv für den pH-Wert des jeweiligen in diesen Verfahren eingesetzten Lösungsmittels sein können. Starke Schwankungen dieses Wertes und besonders ein stark saures Milieu können einen gewissen Verlust spezifischer Lektineigenschaften zur Folge haben. Entsprechend ist die Aufrechterhaltung des vorher bestimmten pH-Bereichs ein notwendiges Merkmal des erfindungsgemäßen Verfahrens. Zur Aufrechterhaltung dieser spezifischen Eigenschaften ist eine pH-Wert-Kontrolle der Lösung in allen Verarbeitungsstufen notwendig, um die Stabilität des Polypeptids zu gewährleisten. In Beispiel 1 ist Verfahren zur Überprüfung des pH-Werts während der Abkühlung, bzw. des Gefrierens von Arzneimittelzubereitungen beschrieben.

In einer bevorzugten Ausführungsform umfaßt das beschriebene Verfahren ein Polypeptid, enthaltend
(a) das rekombinante carbohydratbindende Polypeptid oder ein funktionelles Fragment oder Derivat dieses Polypeptids, welches fusioniert ist mit einem zytotoxisch wirkenden Peptid zu einem Fusionsprotein;
(b) das rekombinante carbohydratbindende Polypeptid oder ein funktionelles Fragment oder Derivat dieses Polypeptids, welches verbunden ist mit einem weiteren Polypeptid, welches eine enzymatische rRNA-N-Glycosidase Aktivität besitzt;
(c) das rekombinante carbohydratbindende Polypeptid oder ein funktionelles Fragment oder Derivat dieses Polypeptids, welches verbunden ist mit einem weiteren Polypeptid, bei dem eine enzymatische rRNA-N-Glycosidase Aktivität durch eine andere zytotoxische Aktivität ersetzt wurde; oder
(d) das rekombinante carbohydratbindende Polypeptid oder ein funktionelles Fragment oder Derivat dieses Polypeptids, welches verbunden ist mit einem Fusionsprotein, umfassend ein Polypeptid mit einer enzymatischen rRNA-N-Glycosidase Aktivität und/oder einer anderen zytotoxischen Aktivität.

Entsprechend dieser bevorzugten Ausführungsform der Erfindung ist das rekombinante carbohydratbindende Polypeptid oder ein funktionelles Fragment oder Derivat dieses Polypeptids an ein weiteres Peptid gebunden, welches eine zytotoxische Aktivität besitzt. Diese Bindung der Peptide kann sowohl kovalent sein, als auch eine Bindung, die auf anderen physiko-chemische Wechselwirkungen beruht. Beispiele für die kovalente Bindung der erfindungsgemäßen Peptide umfassen sowohl Peptidbindungen, die u.a. charakteristisch für Fusionsproteine sind, als auch Disulfid-Bindungen.

Im Sinne der Erfindung ermöglicht das carbohydratbindende Polypeptid oder funktionelle Fragment oder Derivat dieses Polypeptids eine Interaktion des Proteins mit der Zelloberfläche der Zielzelle. Im Anschluß wirkt das Peptid mit zytotoxischer Aktivität entweder direkt an der Zelloberfläche (z.B. durch Bildung von Poren in der Zellmembran) oder nach Aufnahme in die Zelle (z.B. durch Inhibition oder Zerstörung der Proteinbiosynthese, durch Induktion einer Apoptosesignalkaskade oder durch Inhibition oder Zerstörung der Mitochondrienaktivität). Die zytotoxische Aktivität kann durch verschiedene, dem Fachmann bekannte Tests überprüft werden ("JAM-Test" siehe Matzinger (1991), "⁵¹Cr-Freisetzungstest", "Propidiumlodid-Färbung von Zellen" oder "Annexin-V Test" siehe Dulat (2001)).

Beispiele für Peptide mit enzymatischer rRNA-N-Glycosidase Aktivität von Ribosomen-inaktivierenden-Proteinen (RIP's) sind u. a. von Endo et al. (1988 und 1989) und in einem Übersichtsartikel von Peumans et al. (2001) beschrieben.

In einer weiteren bevorzugten Ausführungsform des Verfahrens ist das rekombinante carbohydratbindende Polypeptid die B-Kette eines Ribosomen-inaktivierenden Proteins.

In einer anderen, ebenfalls bevorzugten Ausführungsform ist das weitere Polypeptid, welches mit dem rekombinanten, carbohydratbindenden Polypeptid verbunden ist, die A-Kette eines Ribosomen-inaktivierenden Proteins.

In einer darüber hinaus bevorzugten Ausführungsform entspricht die B-Kette und/oder A-Kette des Ribosomen-inaktivierenden Proteins der B-Kette oder A-Kette eines Ribosomen-inaktivierenden Proteins des Typ II. Dieses Ribosomen-inaktivierende Protein des Typ II ist bevorzugt rViscumin. Sowohl die Funktion, als auch die rekombinante Darstellung des Holoenzyms rViscumin als Beispiel für ein Ribosomen-inaktivierendes Protein ist in EP 0 751 221 B1 beschrieben.

In einer weiter bevorzugten Ausführungsform des Verfahrens wird gewährleistet, daß der pH-Wert der Lösung zwischen 6,0 und 9,0 liegt, weiter bevorzugt ist ein pH-Wert der Lösung zwischen 7,5 und 8,5. Wie in den Beispielen illustriert, ist ein pH von 8,0 besonders bevorzugt. Ein weniger bevorzugter pH Bereich der Lösung ist der Bereich oberhalb von pH 12, da bei diesen höheren pH-Bereichen Desamidierungen zu erwarten sind und sich damit die Eigenschaften des Polypeptids als arzneilicher Wirkstoff ändern würde. Ohne höhere pH-Bereiche auszuschließen ist daher im erfinderischen Verfahren in der Regel ein pH-Bereich von größer als pH 6,0 und kleiner als pH 12 zu wählen. Jedoch kann der Fachmann durchaus auch pH-Bereiche über pH 12 wählen. Es ist dann jedoch bevorzugt, dass der pH-Wert des Arzneimittels vor Verabreichung an den Patienten auf einen physiologischen pH-Bereich eingestellt wird. das Ein Verfahren zur Kontrolle des pH-Werts während der Durchführung des erfindungsgemäßen Verfahrens ist in Beispiel 1 beschrieben.

Ebenfalls bevorzugt ist ein Verfahren, wobei das oder die Salze des Puffersystems in einem Endkonzentrationsbereich von 0,6% bis 2,4% (5 mM bis 200 mM) eingesetzt werden. Weiter bevorzugt ist ein Verfahren, wobei das oder die Salze des Puffersystems in einem Endkonzentrationsbereich von 100 mM bis 200 mM eingesetzt werden. Entsprechend ist z.B. eine Endkonzentration für Tris-Base von 100 bis 200 nM (1,2% bis 2,4%) bevorzugt, da in allen im Zuge dieser Erfindung durchgeführten Studien mit optimierten Formulierungen ein Prozeß-bedingter Verlust von rViscumin von nur 5% beobachtet wurde. Für den Endkonzentrationsbereich von 20 mM bis 100 mM wurde ein entsprechender Verlust im Bereich von 10 bis 15 % detektiert. Wie in den Beispielen gezeigt, wurde für eine Endkonzentration unterhalb der optimalen Konzentration von 20mM ein entsprechender Verlust im Bereich von 10 bis 20% detektiert.

Im Zusammenhang mit dieser Erfindung bedeutet der Begriff "Endkonzentration" die Konzentration der Lösung in Masse/Volumen (mN), die der Fachmann vor dem Abkühlungs-, Einfrierungs-, Sprühtrocknungs- oder Gefriertrocknungs-prozesses einstellt.

Darüber hinaus bevorzugt ist ein Verfahren, wobei das oder die Salze des Puffersystems ausgewählt ist aus der Gruppe umfassend: TRIS/HCl, TRICIN/HCl, HEPES/HCl, Ammoniumcarbonatpuffer, TRIS/Glutaminsäure und TRIS/Asparaginsäure. Wie unter anderem in den angehängten Beispielen beschrieben, gewährleisten diese Puffersysteme für die gewählten Kombinationen von Ausgangsstoffen eine Aufrechterhaltung eines hohen pH-Werts in den entsprechenden Lösungen während einer Gefrierphase. Aus diesem Grunde leisten die entsprechenden Puffersysteme einen entscheidenden Beitrag für die Stabilität des Polypeptids.

In einer anderen bevorzugten Ausführungsform des erfindungsgemäßen Verfahren wird zur Stabilisierung der pharmakologischen Eigenschaften des Polypeptids der Lösung eine oder mehrere, oberflächenaktive Substanzen enthält. Diese oberflächenaktive Substanzen dienen als Netzmittel, setzten folglich die Oberflächenspannung eine Lösung herunter und begünstigen eine Benetzung von Lyophilisates mit einer Rekonstitutionslösung. Darüber hinaus besetzen diese Stoffe sogenannte "hot spots" an den Wandungen der verwendeten Zubereitungsgefäße und Primärpackmittel, an denen beispielsweise rViscumin als hydrophobes Protein bevorzugt gebunden werden kann. In Abwesenheit von Netzmitteln sind Verluste an Protein, bzw. Proteinaktivität während des Herstellungs- und Verpackungsprozesses und in den Arzneimittellösungen wahrscheinlich. Darüber hinaus ist der Zusatz von Netzmitteln von Vorteil, um Verluste nach der Rekonstitution des gefriergetrockneten Pulvers zu vermeiden. Solche Verluste hätten eine ungenaue Dosierung zur Folge.

Bevorzugt werden hierbei nicht-ionische Tenside als oberflächenaktiven Substanzen, wobei diese in einer Endkonzentrationsbereich von 0,01 bis 5,0 % eingesetzt werden.

Bevorzugte nicht-ionische Tenside sind ausgewählt sind aus der Gruppe umfassend: Fettalkohole, Partialglyceride, Polysorbate, Polyoxyethylenfettsäureether und -fettsäureester, Poloxamere (Polyoxypropylen-Polyoxyethylen-Blockpolymere), Saccharidfettsäureester, Polyoxyethylensorbitolether und - fettsäureester, Polyoxyglycerolfettsäureester und Phosphatide.

Bevorzugte Beispiele für Polysorbate sind ausgewählt aus der Gruppe umfassend Polysorbat 80, Polysorbat 20.

Darüber hinaus bevorzugt sind die Polyoxyethylenfettsäureether und -fettsäureester Macrogolether oder Macrogolester, das Poloxamer Pluronic F68, Poloxamer 166 oder 188 und die Phosphatide, wie z.B. Lecithine. In diesem Zusammenhang sind auch Derivate von Lecithinen aus Soja- oder Hühnereiweiss umfaßt.

Ebenfalls bevorzugt sind als oberflächenaktiven Substanzen sind amphotere Tenside, die in einer Endkonzentrationsbereich von 0,01 bis 5,0 % eingesetzt werden.

In einer ebenfalls bevorzugten Ausführungsform des erfindungsgemäßen Verfahrens werden der Lösung für eine Gefriertrocknung ein oder mehrere Lyoprotektoren in einem Endkonzentrationsbereich von 4,0 bis 10 % und Kryoprotektoren in einem Endkonzentrationsbereich von 0,01 bis 1,0 % zugesetzt. Lyoprotektoren dienen in diesem Zusammenhang dem Schutz der Substanzen bei der Trocknung, Kryoprotektoren haben eine entsprechende Aufgabe während des Gefrierens. Die hier angegebenen Endkonzentrationsbereiche für den Einsatz von Lyoprotektoren und/oder Kryoprotektoren gelten als bevorzugt, folglich sind durch das erfindungsgemäß Verfahren auch Endkonzentrationsbereiche umfaßt, die außerhalb der bevorzugten Endkonzentrationsbereichs liegen. Die Lyoprotektoren werden in einem Endkonzentrationsbereich von 4,0 bis 10 % eingesetzt. In Kombination mit den Lyoprotektoren oder auch in deren Abwesenheit werden die Kryoprotektoren bevorzugt in einem Endkonzentrationsbereich von 0,05 bis 0,1 % der Lösung zugesetzt.

Vorzugsweise werden Dextrane mit einem molekularen Masse von 1000 bis 100000 Da und besonders bevorzugt von 1000 - 10000 Da verwendet. Wie in den hierin beschriebenen Beispielen dokumentiert, stellen Dextrane bevorzugte Lyoprotektoren dar, die vorzugsweise ohne Mannit verwendet werden können, jedoch durchaus auch zusammen mit anderen Lyoprotektoren im erfinderischen Verfahren genutzt werden können. Wie die Beispiele zeigen, können Dextrane vorzugsweise auch alleine (ohne weitere) Lyoprotektoren im erfinderischen Verfahren eingesetzt werden. Die alleinige Eignung von Dextranen als Lyoprotektoren im erfinderischen Verfahren, insbesondere der Gefriertrocknungsprozess ist überraschend, da im Stand der Technik beschrieben ist, dass Dextran nur als begleitender Hilfsstoff einen Beitrag zur Stabilisierung von Proteinen zu leisten vermag ( Carpenter et al. 1993, Carpenter et al. 1999, Allison et al. 1999, Allison et al. 2000)

Ebenfalls bevorzugt werden als Kryoprotektoren ionische Substanzen verwendet. Diese ionischen Substanzen werden wiederum bevorzugt ausgewählt aus der Gruppe umfassend Natriumchlorid, Natriumsulfat, Kaliumchlorid und Kaliumsulfat. Ebenfalls erfindungsgemäß ist die Verwendung von Natriumsalzen der Edetinsäure. Diese Salze tragen durch Komplexierung von im Verlauf des Herstellungsprozesses eingetragenen Metallkationen zu einer weiteren Stabilisierung der Polypeptide bei.

Entsprechend des erfindungsgemäßen Verfahrens bilden die Lyoprotektoren und Kryoprotektoren bei der Gefriertrocknung amorphe Strukturen. Diese Lyoprotektoren und Kryoprotektoren verhindern, daß es während des Gefriertrockunungsvorgangs zur Ausbildung von Kristallgittern (konstante Abstände von Atomen in einem Feststoff) kommt. Die Abwesenheit kristalliner Strukturen in einem Feststoff kann durch eine Pulverkristallstrukturanalyse (z.B. durch Beugung von Röntgenstrahelen) nachgewiesen werden.

In einer weiteren bevorzugten Ausführungsform des Verfahrens werden Aminosäuren als Stabilisatoren eingesetzt. Vorzugsweise werden diese in einer Konzentration von 0,01 bis 50 mg/ml eingesetzt. Zusätzlich zur stabilisierenden Eigenschaft können Aminosäuren selbst erfindungsgemäß auch als Puffersubstanzen eingesetzt werden.

Weiter bevorzugt werden die Aminosäuren ausgewählt aus der Gruppe umfassend saure Aminosäuren wie Glutaminsäure und Asparaginsäure, die basische Aminosäure Arginin und die neutrale Aminosäure Valin.

In einer weiteren Ausführungsform des erfinderischen Verfahrens wird das Polypeptid, das mindestens ein rekombinantes carbohydratbindendes Polypeptid oder ein funktionelles Derivat oder ein Fragment des rekombinanten carbohydratbindenden Polypeptides umfasst, in einer Endkonzentration von 0,000001% (10ng/ml) bis 1,0% (10 mg/ml) eingesetzt. Besonders bevorzugt ist hierbei eine Proteinkonzentration von 0,00001% (100ng/ml) bis 0,1% (1mg/ml).

Eine ebenfalls bevorzugte Ausführungsform des Verfahrens umfassend die Weiterformulierung oder Rekonstitution des Arzneimittels als wäßrige oder nicht-wäßrige Lösung. Dies schließt darüber hinaus die Weiterformulierung des Arzneimittels als Injektions-, Instillations- oder Infusionslösung ein. Erfindungsgemäße Injektionslösungen werden, abhängig von dem zu therapierenden Leiden oder Krankheit subcutan, intramuskulär, intravenös, intrakardial oder intraperitoneal verabreicht. Lösungen zur Instillation in eine Körperhöhle werden abhängig von dem zu therapierenden Leiden z.B. in die Harnblase instilliert.

In einer weiteren bevorzugten Ausführungsform des Verfahrens ist darüber hinaus die Weiterformulierung oder Rekonstitution des Arzneimittels für gastrointestinale, orale, nasale, pulmonale, dermale, transdermale oder lokale Anwendungen umfaßt.

Weiter bevorzugt ist die Weiterformulierung des Arzneimittels zu Saft, Kapseln, Tabletten, Zäpfchen oder Gelen.

Die genannten Gele, welche durch Weiterformulierung des erfindungsgemäßen Arzneimittels hergestellt werden, können durch die Verwendung von anorganische und organische Hydrogelbildner gemeinsam mit wäßrigen oder wäßrig/alkoholischen Lösungen erhalten werden. Hierbei umfaßt sind Gelbildner natürlichen, teilsynthetischen und synthetischen Ursprungs. Gemeinsam ist diesen Molekülen eine z.T. extreme Quellbarkeit, die zur Ausbildung von streichbaren Gelen führt.

Ebenfalls bevorzugt ist darüber hinaus die Weiterformulierung des Arzneimittels zu einem Pulver zur Inhalation, welches in einem Inhalator verabreicht wird.

Die Erfindung betrifft darüber hinaus ein Arzneimittel, welches nach einem der erfindungsgemäßen Verfahren hergestellt wird.

Ebenfalls betrifft die Erfindung die Verwendung eines Polypeptids zur Herstellung eines solchen Arzneimittels.

Die Verabreichung des erfindungsgemäßen Arzneimittels kann, abhängig von den beschriebenen Weiterformulierungen, auf verschiedenen Wegen erfolgen, z.B. intravenös, intraperitoneal, subcutan, intramuskulär, lokal oder intradermal. Die Art der Dosierung wird vom behandelnden Arzt entsprechend den klinischen Faktoren bestimmt. Es ist dem Fachmann bekannt, daß die Art der Dosierung von verschiedenen Faktoren abhängig ist, wie z.B. der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden.

Die Figuren zeigen
**Figur 1** In Figur 1 ist die Änderung des pH-Werts einer Pufferlösung in Abhängigkeit von der Temperatur dargestellt. Die Pufferlösung entspricht einem 20 mM Phosphatpuffer und enthält darüber hinaus 0,1 % Natriumchlorid. Diese Pufferlösung wurde, wie in Beispiel 1 beschrieben, in einem handelsüblichen Kryostat mit Temperatursteuerung abgekühlt. Der pH-Wert in der Lösung wurde mit speziell geeigneten pH-Elektroden bestimmt. Die Abkühlrate betrug im dargestellten Versuch 1,2 K. Der Verlauf der dargestellten Kurve zeigt, daß das Abkühlen der Pufferlösung von RT auf den Gefrierpunkt der Lösung keinen signifikanten Einfluß auf den pH-Wert dieser Lösung hat. Wird die Lösung auf Temperaturen unterhalb deren Gefrierpunkt abgekühlt, so ist ein deutliches Absinken des pH-Werts von 8 auf unter 5 zu beobachten.

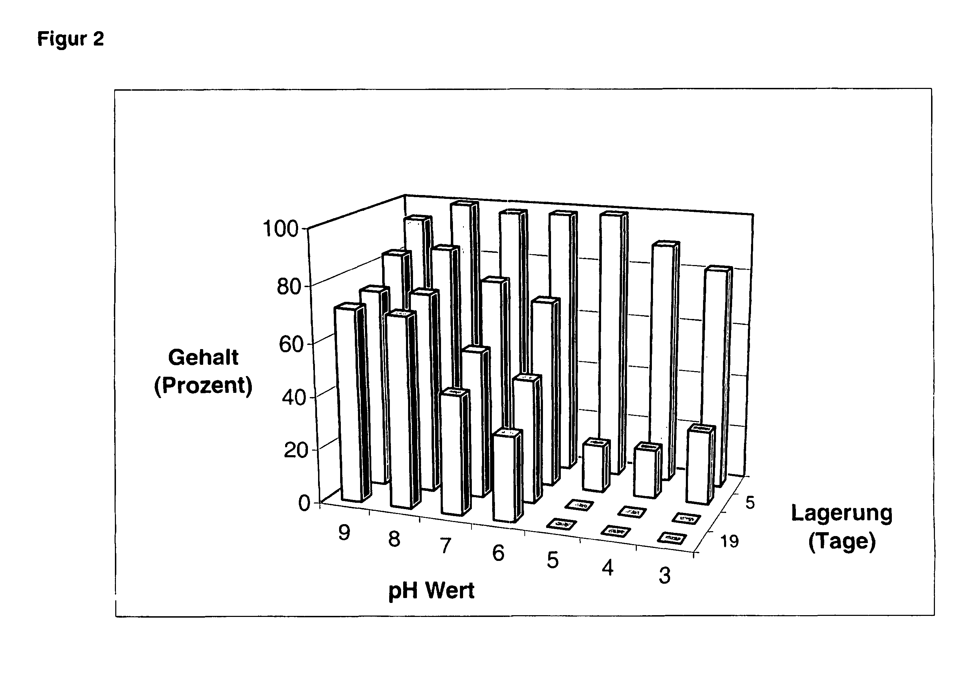

**Figur 2** In Figur 2 ist die Stabilität von carbohydrat-spezifischen rViscumin in Abhängigkeit vom pH-Wert und Kurzzeitlagerung bei 2 - 8 °C, rViscumin in gepufferter saliner Lösung dargestellt.
   Die Pufferlösung entspricht einem 20 mM Phosphatpuffer (pH 7,2), die mit NaOH (1 M und 0,1 M) bzw. HCl (10 % bzw. 1 %) auf pH-Werte von 3, 4, 5, 7, 8 und 9 eingestellt wurde. Die phosphatgepufferten Lösungen enthalten darüber hinaus NaCl in einer Konzentration von 0,7 bis 0,9 % zur Einstellung der Isotonie der Lösungen und nieder molekulares Polyvinylpyrrolidon in einer Konzentration von 0,1 g/l zur Vermeidung einer Adsorption des Polypeptids an die Gefäßoberfläche.
   In dem in der Abbildung dargestellten Versuch wurde beobachtet, daß die Stabilität des Polypeptids rViscumin in den gepufferten Lösungen mit sinkendem pH-Wert stark abnimmt. Unterhalb eines pH-Werts von pH 6 ist bereits nach kurzer Lagerdauer kein rViscumin mit carbohydrat-spezifischen Eigenschaften vorhanden.

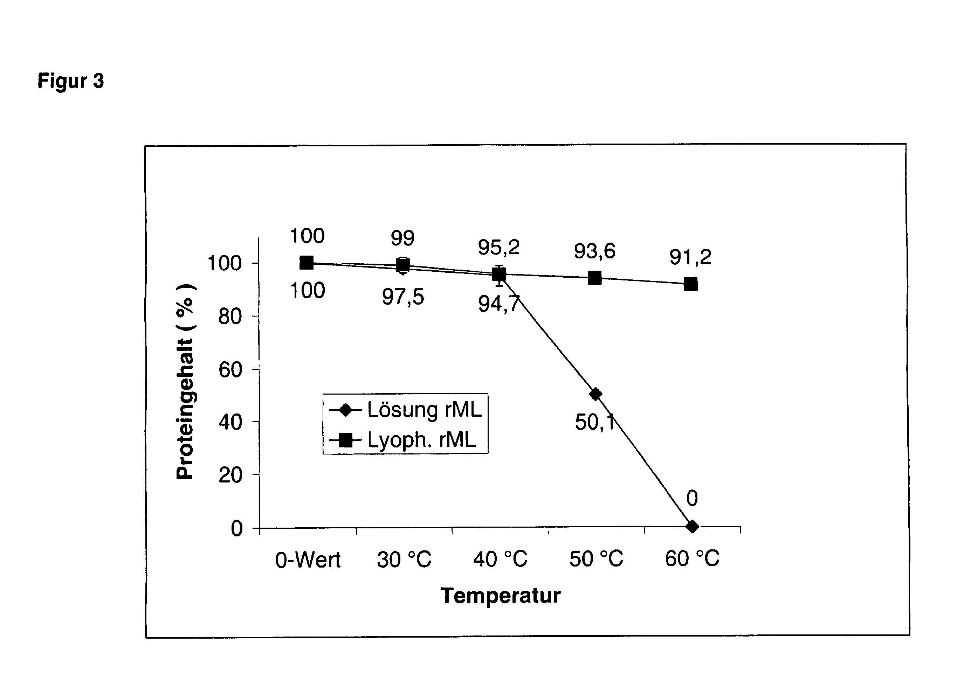

**Figur 3** In Figur 3 ist die Stabilität von carbohydrat-spezifischen rViscumin (rML) in gepufferter stabilisierter Lösung und dem daraus hergestelltem gefriergetrocknetem Pulver (Lyophilisat) in Abhängigkeit von der Temperatur dargestellt.
   Die Pufferlösung entspricht einem 200 mM Tris/HCl Puffer (pH 8,0), die 8,0 % (w/v) Dextran T10, 0,1 % (w/v) NaCl und 0,1 % (w/v) Polysorbat 80 enthält. rViscumin ist in einer Konzentration von 2,0 µg/ml in der Lösung enthalten. Die Lösung wird entsprechend des in Beispiel 3 beschriebenen Versuchs aufgeteilt, behandelt und untersucht.
   Das in der Abbildung dargestellte Versuchsergebnis zeigt, daß der Gehalt an rViscumin in der gepufferten, stabilisierten Lösung ab einer Temperatur von 40°C stark abnimmt. Bei 50°C werden nur noch 50 % der Ausgangskonzentration des rViscumin mit carbohydrat-spezifischen Eigenschaften nachgewiesen. Bei 60°C wird kein carbohydrat-spezifisches rViscumin mehr detektiert. Die Zersetzungstemperatur von rViscumin in Lösung ist damit zwischen 40°C und 50°C anzusetzen.
   Der detektierte Gehalt an rViscumin mit carbohydrat-spezifischen Eigenschaften im Festkörper nimmt nur sehr langsam mit der Erhöhung der Temperatur ab. Bei einer Temperatur von 50°C kann noch ein Gehalt von 94 % und bei 60°C ein Gehalt von 91 % des Ausgangsgehalts nachgewiesen werden.
**Figur 4**

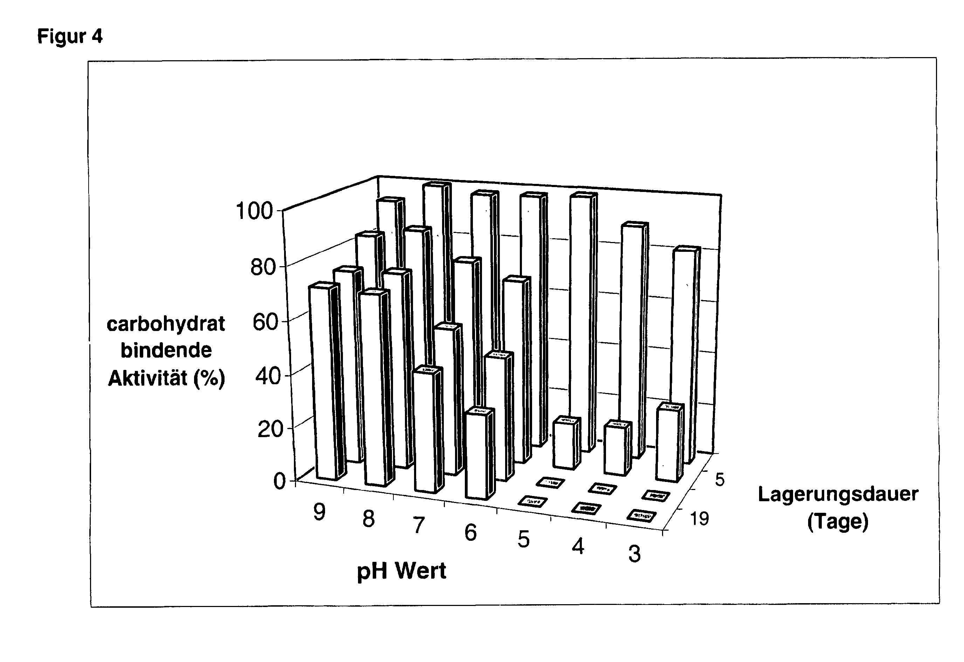

   Figur 4 veranschaulicht die Abhängigkeit der Stabilität der carbohydrat bindenden Aktivität von rViscumin in wässriger Lösung mit Änderung des pH Wertes.
**Figur 5**

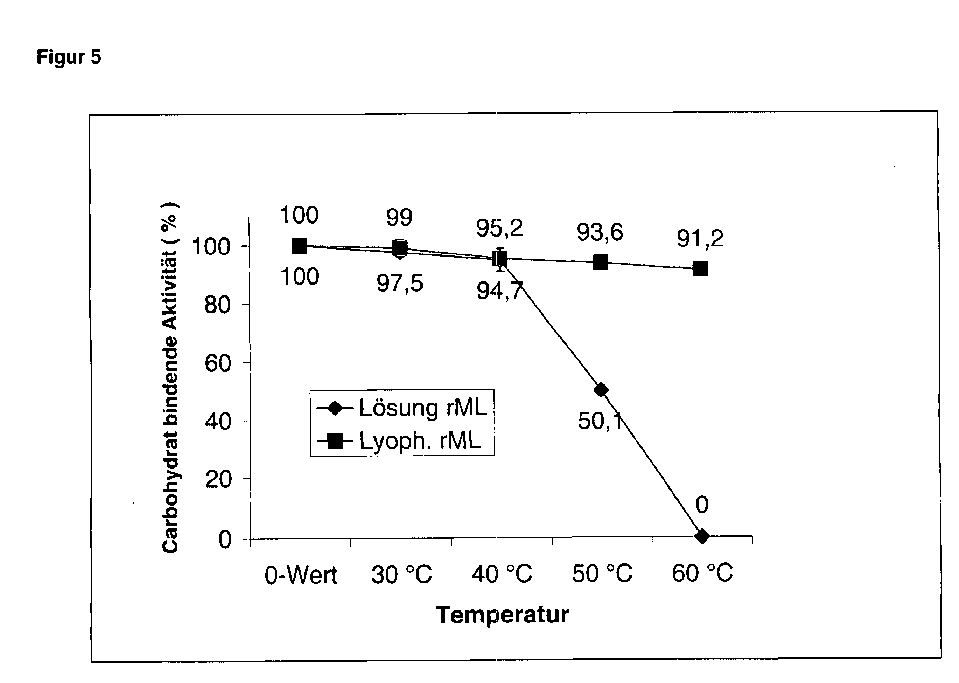

   Figur 5 veranschaulicht die Abhängigkeit der carbohydrat bindenden Aktivität von rViscumin in wässriger Lösung und als gefriergetrocknetes Pulver mit zunehmender Temperatur
**Figur 6**

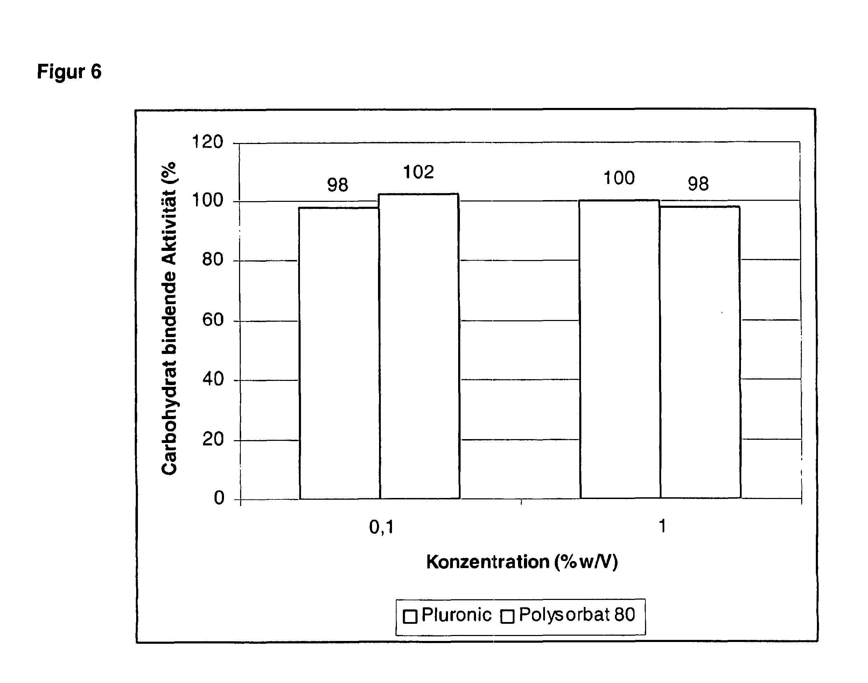

   Figur 6 zeigt den Einfluss, den die Hilfsstoffe Pluronic F68 und Polysorbat 80 in ihrer Eigenschaft als Kryoprotektoren auf den Prozessschritt Frieren/Auftauen einer wässrigen Lösung von rViscumin in 100 mM TRIS Puffer pH 8,0 haben. Die Lösung enthalten den Lyoprotektor Dextran T1 in einer unterhalb des bevorzugten Bereichs liegenden Konzentration von 2 %.
**Figur 7**

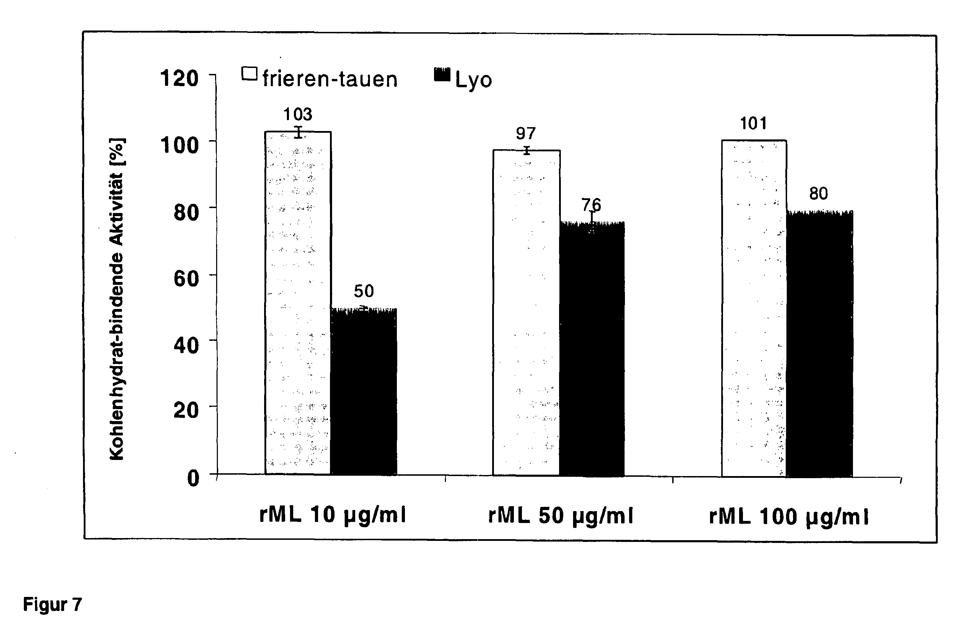

   Figur 7 zeigt den Einfluss, den die Proteinkonzentration einer wässrigen Lösung von rViscumin auf den Gefriertrockungsprozess hat.
**Figur 8**

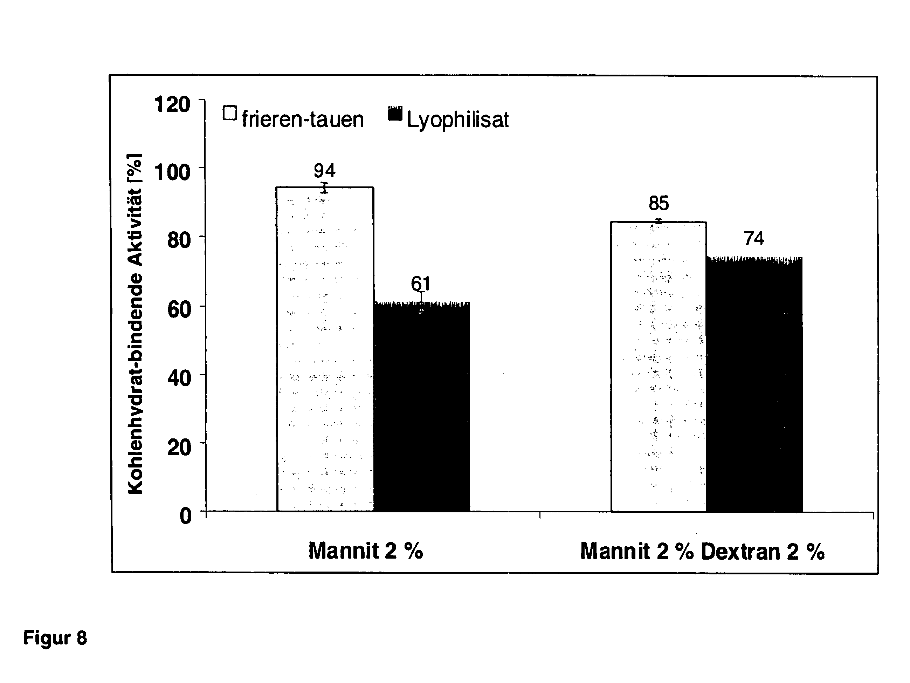

   Figur 8 zeigt den Einfluss, den Lyoprotektor Mannit und eine Mischung von Mannit gemeinsam mit einem nicht kristallisierenden Lyoprotektor auf rViscumin hat.
**Figur 9**

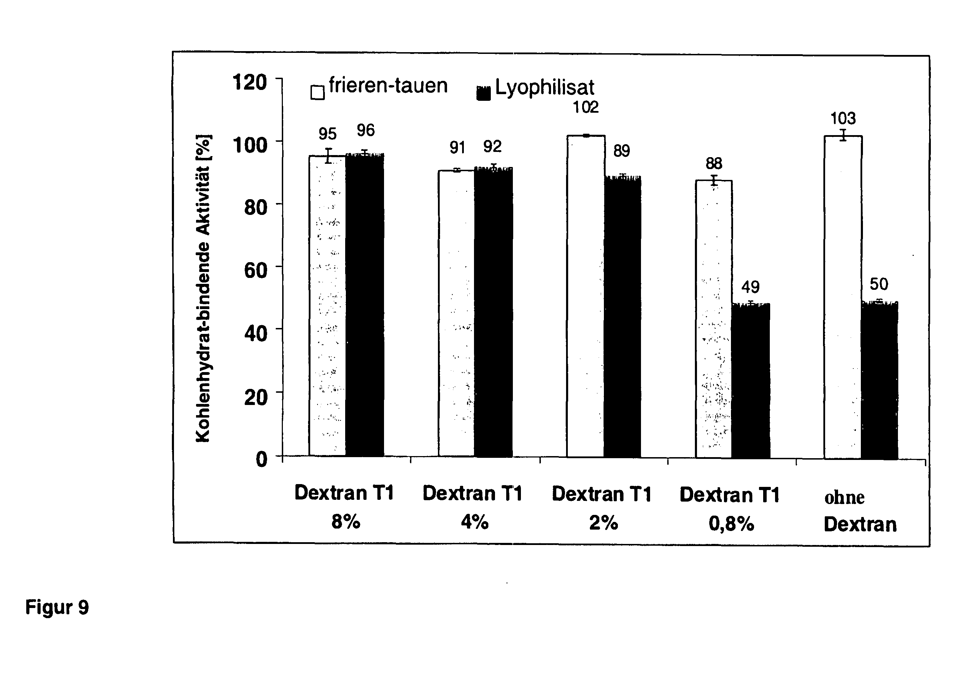

   Figur 9 zeigt die Eignung und den optimalen Bereich des Lyoprotektors Dextran T1 auf die Stabilität von rViscumin während der Gefriertrocknung.
**Figur 10**

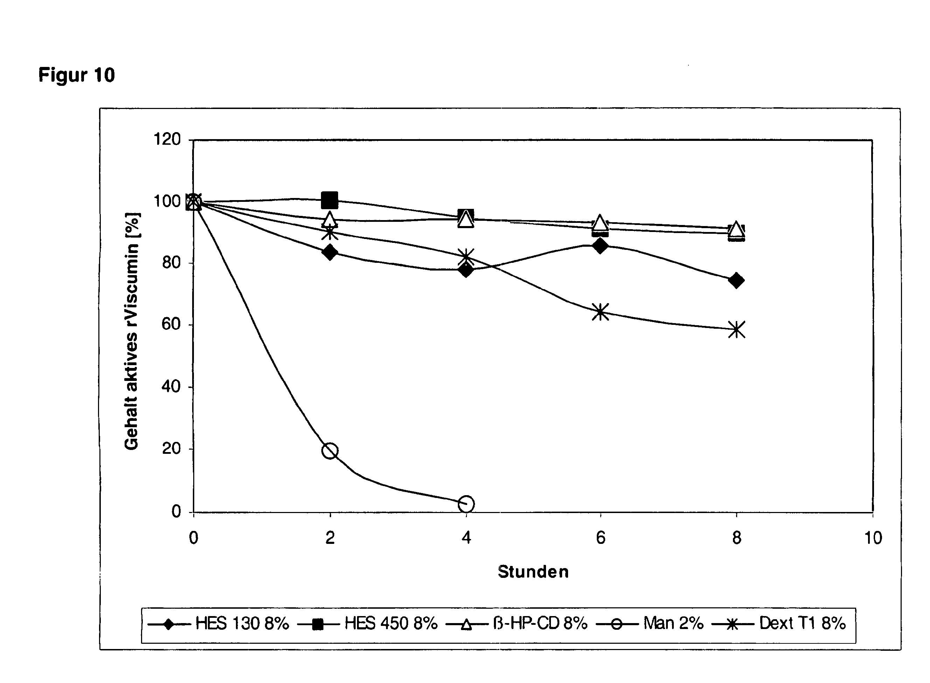

   Figur 10 zeigt den Einfluss verschiedener Lyoprotektoren auf die Stabilität von gefriergetrockneten rViscumin-Zubereitungen bei erhöhter Temperatur von 60 Grad C.
**Figur 11**

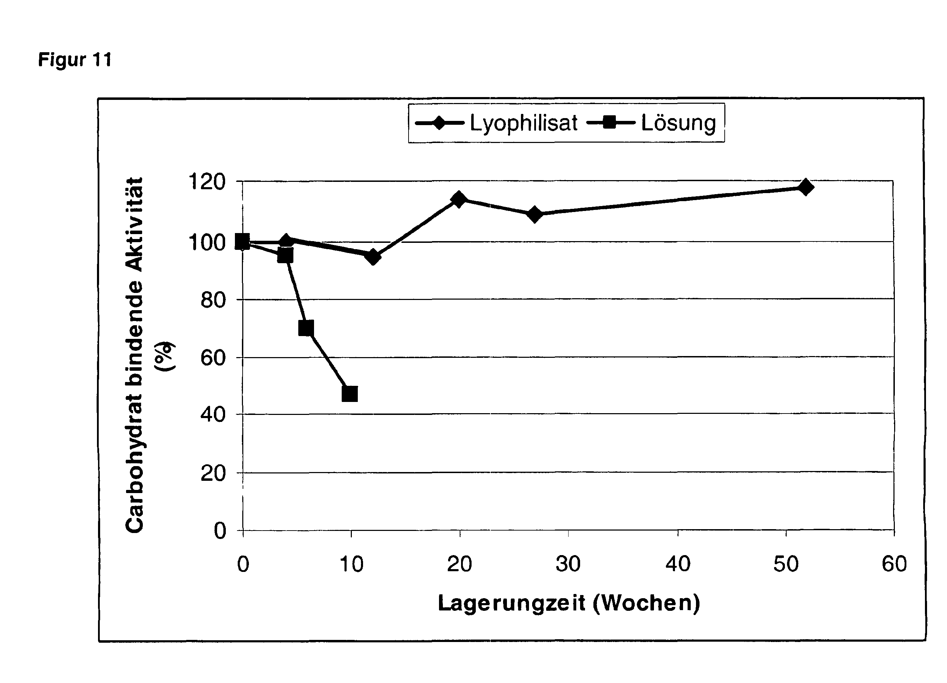

   Figur 11 zeigt die Lagerungsstabilität einer wässrigen rViscumin-Zubereitung (Quadrate) über 10 Wochen und eines Lyophilisats (Rauten) über 56 Wochen bei einer Lagerungstemperatur von 2-8 Grad C.

### Referenz beispiel 1:

### Verfahren zur Überprüfung des ph-Werts während der Abkühlung, bzw. des Gefrierens von Arzneimitteln

rViscumin ist ein dimeres rekombinant hergestelltes pflanzliches Protein mit zuckerspezifischen Bindeaktivitäten. Die pharmakologische Wirkung des Proteins, Auslösung von Apoptose von Zellen, korreliert mit dem Erhalt der zuckerspezifischen Bindeaktivität. Der Erhalt der Zuckerspezifität ist stark abhängig von dem pH-Wert des umgebenden Mediums. Mit sinkendem pH-Wert des Mediums nimmt bei einem pH-Wert von kleiner als 6,0 die zuckerbindende Aktivität von rViscumin stark ab. Dies trifft auch zu bei einer pH-Veränderung während des Einfriervorgangs bei der Gefriertrocknung von wässrigen Zubereitungen mit rViscumin. Aus diesem Grund ist die pH-Kontrolle von wässrigen Puffersystemen während des Gefrierens von rViscumin-Arzneimittelzubereitungen im Rahmen der Gefrietrocknung notwendig.

Die Aufgabe kann gelöst werden, indem pharmazeutische Zubereitungen von rViscumin oder dessen Basisrezeptur ohne Wirkstoff (Kombination der Puffersalze) in einem Volumen von 15 ml in üblichen Gefrierflaschen (Vials) hergestellt werden. Die Gefrierflaschen werden in einen handelsüblichen Kryostaten mit kontrollierter Temperatursteuerung gestellt. Spezielle geeignete pH-Elektroden (z.B. die druckresistente Sure-Flow pHuture Probe mit Konverter Model 605 - Spannungsversorgung für ISFET-Elektroden, Orion- oder die frostresistente Glaselektrode, Schott Geräte GmbH, Hofheim) werden zur pH-Messung verwendet. Die Aufzeichnung der pH-Werte erfolgt mit handelsüblichen pH-Metern. Eine Abkühlrate von 1,2 K ist geeignet, die Simulation der Kühlrate von Gefriertrocknungsgeräten abzubilden. Die pH-Werte in der Lösung werden in Abhängigkeit von der Temperatur gemessen.

In Figur 1 ist der von der Temperatur abhängige Verlauf des pH-Werts eines 20mM Natriumphosphatpuffers (pH 8,0 bei RT) dargestellt.

Phosphatpuffer zeigen mit Erniedrigung der Temperatur unterhalb von 0°C eine sprunghafte und starke Erniedrigung des pH-Wertes, wie am Beispiel des 20mM Phophatpuffers mit 0,1% (w/v) Natriumchlorid, gemessen mit dem beschriebenen Verfahren, belegt werden kann. Dies läßt auf eine physikalische Veränderung des Puffersystems schließen. Es ist bekannt, daß Natriummonohydrogenphosphat mit sinkender Temperatur aus wäßrigen phosphatgepufferten Lösungen bevorzugt kristallisiert und damit diese pH-Veränderung bedingt.

Wässrige Zubereitungen von rViscumin wurden bereits in EP 0 751 221 B1 beschrieben. Diese als Arzneimittel geeignete wässrige Zubereitungen sind wässrige Phosphat gepufferte Lösungen pH 7,2 und einer rViscumin Konzentration von 100 - 200 ng/ml und haben zum Beispiel folgende Zusammensetzung:

| | |
|---|---|
| rViscumin | 100 ng |
| Natriummonohydrogenphosphat Dihydrat | 3,56 mg |
| Natriumdihydrogenphosphat Dihydrat | 0,64 mg |
| Natriumchlorid | 67,0 mg |
| Poly(1-vinyl-2-pyrrolidon) K 17 | 0,5 mg |
| Wasser zur Injektion | ad 1 ml. |

Phosphat Puffer pH 7,2 zeigen bei Abkühlung und Einfrieren eine mit sinkender Temperatur bedingte Abnahme des pH Wertes wie sie auch für Phosphat Puffer pH 8.0 gemessen wurde und wie sie in Figur 1 beschrieben ist. Es ist dem Fachmann bekannt, dass niedrige Anfangswerte des pH bei Gefrieren der wässrigen Lösung zu stärkeren Verschiebungen in den sauren Bereich führen, da die Konzentration an Natriumdihydrogenphosphat in der Lösung erhöht ist. Werden Zubereitungen der oben genannten Zusammensetzung gefriergetrocknet, führt dies zwangsläufig zu niedrigen pH Bedingungen unterhalb von pH 6, unter denen rViscumin nicht stabil ist und es kommt durch Denaturierung des Proteins zu Aktivitätsverlusten, wie es für wässrige rViscumin Zubereitungen in Figur 5 veranschaulicht ist.

Die pH-Verläufe der biologischen Puffer TRIS/HCl, TRICIN/HCl und Hepes/HCl pH 8,0 werden in Gloger O., Müller B.W., 2000 gezeigt und diskutiert.

Puffersysteme bestehend aus TRIS/HCl, TRISIN/HCl und Hepes/HCl eingestellt auf den pH-Wert 8,0 zeigen mit Erniedrigung der Temperatur eine kontinuierliche geringe pH-Veränderung zu größeren pH-Werten bis zu 9,0 (Gloger O., Müller B.W., 2000).

### Referenz beispiel 2: Stabilität von carbohydrat-spezifischen rViscumin in Abhängigkeit vom pH-Wert und Kurzzeitlagerung

rViscumin wird in einer Konzentration von 200ng/ml in verschiedenen Puffern gelöst. Ausgehend von einem 20 mM Phosphatpuffer (pH 7,4) werden mit NaOH (1 M und 0,1 M) bzw. HCl (10 % bzw. 1 %) Puffer der pH-Werte 3, 4, 5, 7, 8 und 9 hergestellt. Die phosphatgepufferten Lösungen enthalten darüber hinaus NaCl in einer Endkonzentration von 0,7 - 0,9 % zur Einstellung einer Isotonie der Lösung und nieder molekulares Polyvinylpyrrolidon in einer Konzentration von 0,1g/l zur Vermeidung einer Adsorption des Polypeptids an die Gefäßoberfläche. Die Lösungen werden über eine Membran (Porengröße 0,2 µm) keimfiltriert und in geschlossenen Polyethylengefäßen unter kontrollierten Temperaturbedinungen bei 2 - 8°C gelagert. In Abhängigkeit von der Zeit werden Proben genommen. Diese Proben werden 1:10 mit 20 mM Phosphatpuffer (pH 7,4) verdünnt um einheitliche Lösungen zur Bestimmung des Porteingehalts mit Lektinaktivität mittels eines spezifischen Enzym-gekoppelten Immunoassays unter Verwendung eines Glykoprotein und eines spezifischen monoklonalen Antikörpers zu erhalten. Ein Beispiel für einen Assay zur Bestimmung des Proteingehalts einer Lösung mit Lektinaktivität ist in Beispiel 4 beschrieben.

Der in Figur 2 dargestellte Versuch zeigt, daß die Stabilität des Polypeptids rViscumin in den gepufferten Lösungen mit sinkendem pH-Wert stark abnimmt. Unterhalb eines pH-Werts von pH 6 ist nach kurzer Lagerdauer kein rViscumin mit Lektinaktivität mehr in den Lösungen enthalten. Die höchste Stabilität von rViscumin unter Erhalt der Lektinaktivität wird bei hohen pH-Werten beobachtet.

### Referenz beispiel 3: Stabilität von rViscumin (rML) Lyophilisat

rViscumin ist in einer Konzentration von 2,0 µg/ml in einer gepufferten, stabilisierten Lösung, die 200 mM Tris/HCl Puffer (pH 8,0), 8,0 % (w/v) Dextran T10, 0,1 % (w/v) NaCl und 0,1 % (w/v) Polysorbat 80 enthält, gelöst. Ein Teil dieser Lösung wird unter aseptischen Bedingungen mittels Gefriertrocknung in ein Pulver überführt. Hierfür werden 0,5 ml der Lösung nach Keimfiltration über einen 0,2 µm Filter in Glasvials gefüllt, mit einem Lyophilisationsstopfen teilverschlossen und in einer Lyophilisationsanlage getrocknet. Der andere Teil wird ebenfalls keimfiltriert, in Glasvials gefüllt und verschlossen bis zur Untersuchung bei 2 - 8°C gelagert.

Nach der Gefriertrocknung werden sowohl die Glasvials mit der wäßrigen Lösung, als auch jene mit dem Festkörper (getrocknete Lösung) in ein kontrolliertes Wasserbad mit Temperatur- und Zeitsteuerung gegeben. Die Glasvials wurden folgenden Temperaturen ausgesetzt:
5 Minuten bei 30°C
heizen mit Temperaturzunahme von 1,5°C/Minute
5 Minuten bei 40°C
heizen mit Temperaturzunahme von 1,5°C/Minute
5 Minuten bei 50°C
heizen mit Temperaturzunahme von 1,5°C/Minute
5 Minuten bei 60°C

Der Proteingehalt mit Lektinaktivität in den ausgewählten Proben der Lösung und des Festkörpers wurde nach der Temperaturführung mittels eines spezifischen Enzym-gekoppelten Immunoassays unter Verwendung eines Glykoprotein und eines spezifischen monoklonalen Antikörpers bestimmt. Ein Beispiel für einen Assay zur Bestimmung des Proteingehalts einer Lösung mit Lektinaktivität ist in Beispiel 4 beschrieben.

Der in Figur 3 dargestellte Versuch zeigt, daß der Gehalt an rViscumin in der gepufferten stabilisierten Lösung ab einer Temperatur von 40 °C stark abnimmt. Bei 50°C werden nur noch 50 % der Ausgangskonzentration von rViscumin mit Lektinaktivität detektiert. Nachdem die Lösung auf 60°C erhitzt wurde, kann kein rViscumin mit Lektinaktivität mehr gefunden werden. Die Zersetzungstemperatur von rViscumin in Lösung ist demzufolge zwischen 40 °C und 50 °C anzusetzen. Der Gehalt an rViscumin mit Lektinaktivität im Festkörper nimmt nur sehr langsam mit der Erhöhung der Temperatur ab. Bei einer Temperatur von 50°C wird ein Gehalt von 94 % und bei 60°C ein Gehalt von 91 % des Ausgangsgehalts an rViscumin mit Lektinaktivität gefunden. Dies zeigt, daß rViscumin im lyophilisierten Pulver wesentlich stabiler ist, als in der Lösung.

### Referenz beispiel 4: Bestimmung des Proteingehalts einer Lösung mit Lektinaktivität

100 µl einer Lösung von 0,1 mg/ml Asialofetuin in Carbonatpuffer pH 9,6 werden in die Löcher einer 96 Mikrotiterplatte mit hoher Proteinbindung gegeben und 16 Stunden bei Umgebungstemperatur inkubiert. Nach dreimaligem Waschen mit PBS enthaltend 0,05 g/l Polysorbat 80 werden die Löcher der Mikrotiterplatte für 1 Stunde bei Umgebungstemperatur mit 200 µl PBS enthaltend 10 g/l Rinderserumalbumin und 0,05 g/l Polysorbat 80 inkubiert (Blockieren unspezifischer Bindungsstellen). Nach dreimaligen Waschen wird jeweils 100 µl der rViscumin Referenzlösung im Konzentrationsbereich 10 - 200 ng/ml, 100 µl der Prüflösung und für Ermittlung des Leerwerts 100 µl des Puffers (PBS mit 0,05 g/l Polysorbat 80) in die Löcher gegeben und für zwei Stunden bei Umgebungstemperatur inkubiert. Danach werden die Löcher der Mikrotiterplatte gewaschen und mit 100 µl einer Lösung eines spezifischen monoklonalen Detektionsantikörper (anti-rViscumin A-Kette IgG der Maus) der Konzentration 1 µg/ml in PBS enthaltend 0,05 g/l Polysorbat 80 und 0,1 g/l Rinderserumalbumin versetzt und für eine Stunde bei Umgebungstemperatur inkubiert. Die Löcher der Mikrotiterplatten werden dreimal gewaschen und mit 100 µl eines spezifischen handelsüblichen Peroxidase (POD) gekoppelten anti-IgG-Maus Antikörper in der Verdünnung entsprechend den Angaben des Lieferanten versetzt und für 1 Stunde bei Umgebungstemperatur inkubiert. Die Löcher der Mikrotiterplatte werden sechsmal gewaschen und anschließend mit 100 µl einer Lösung einer handelsüblichen ortho-Phenylendiammin/H₂O₂-Tablette in 25 ml Citratpuffer pH 5 versetzt und 15 Minuten bei Umgebungstemperatur im Dunkeln inkubiert. Nach 15 Minuten wird zu jedes Loch 100 µl einer 1 M Schwefelsäure gegeben und die Stärke der Färbung der Lösung durch Absorptionsmessung bestimmt.

Der Gehalt in den Prüflösungen wird im Vergleich zu den Referenzlösungen ermittelt.

### Beispiel 5: Dextran enthaltende rViscumin-Injektionslösung 10 µg/ml (Lyophilisat)

Im folgenden sind verschiedenen Rezepturen für Dextran enthaltende Injektionslösungen beschrieben.

Hierfür wurden Polysorbat, Tris-Base und Dextran in 80 % der benötigten Menge Wasser für Injektionszwecke gelöst. Anschließend wird der pH mit HCl (1 N) auf 8,0 eingestellt. In diese Lösung wird das rViscumin eingebracht und gut durchmischt. Mit dem restlichen Wasser wird auf das geforderte Sollvolumen ergänzt. Anschließend wird die Lösung über einen 0,2 µm Filter sterilfiltriert. Die Lösung wird unter aseptischen Bedingungen in Glasvials eingefüllt, mit dem Lyophilisationsstopfen vorverschlossen und in der Gefriertrocknungsanlage getrocknet.

| Rezeptur mit Dextran T1 | |
|---|---|
| rViscumin | 0,01 mg |
| Polysorbat 80 | 1 mg |
| Tris-Base | 24,2 mg |
| HCl (1 N) | q.s. pH 8,0 |
| Dextran T 1 | 80 mg |
| Wasser für Injektionszwecke | ad 1,0 ml |

Im Folgenden ist darüber hinaus die Zubereitung einer rViscumin-Injektionslösung beschrieben, die zusätzlich NaCl umfaßt. Dieses wird gleichzeitig mit Polysorbat, Tris-Base und Dextran im Wasser gelöst.

| Rezeptur mit Dextran T1 und NaCl | |
|---|---|
| rViscumin | 0,01 mg |
| Polysorbat 80 | 1 mg |
| Tris-Base | 24,2 mg |
| HCl (1 N) | q.s. pH 8,0 |
| NaCl | 1 mg |
| Dextran T 1 | 80 mg |
| Wasser für Injektionszwecke | ad 1,0 ml |

Im letzten Beispiel für diese Gruppe von rViscumin-Zubereitungen ist des weiteren die Zubereitung einer rViscumin-Injektionslösung beschrieben, die zusätzlich zum NaCl auch Na-EDTA umfaßt. Diese werden gleichzeitig mit Polysorbat, Tris-Base und Dextran im Wasser gelöst.

| Rezeptur mit Dextran T1, NaCl und Na-EDTA | |
|---|---|
| rViscumin | 0,01 mg |
| Polysorbat 80 | 1 mg |
| Tris-Base | 24,2 mg |
| HCl (1 N) | q.s. pH 8,0 |
| Dinatrium-EDTA | 0,01 mg |
| NaCl | 1 mg |
| Dextran T 1 | 80 mg |
| Wasser für Injektionszwecke | ad 1,0 ml |

Für die Rekonstitution der Lyophilisate werden diese in den dargestellten Beispielen jeweils in der angegebenen Wassermenge aufgenommen.

### Referenz beispiel 6: β-HP-Cyclodextrin enthaltende rViscumin-Injektionslösung 10 µg/ml (Lyophilisat)

| Rezeptur mit ß-HP-Cyclodextrin | |
|---|---|
| rViscumin | 0,01 mg |
| Polysorbat 80 | 1 mg |
| Tris-Base | 24,2 mg |
| HCl (1 N) | q.s. pH 8,0 |
| Dinatrium-EDTA | 0,01 mg |
| β-HP-Cyclodextrin | 80 mg |
| Wasser für Injektionszwecke | ad 1,0 ml |

Für die Herstellung dieser Injektionslösung werden Polysorbat, Tris-Base, Dinatriumedetinsäure und β-Hydoxypropyl-Cyclodextrin in 80 % der benötigten Menge Wasser für Injektionszwecke gelöst. Anschließend wird der pH mit HCl (1 N) auf 8,0 eingestellt. In diese Lösung wird das rViscumin eingebracht und gut durchmischt. Mit dem restlichen Wasser wird auf das geforderte Sollvolumen ergänzt. Anschließend wird die Lösung über einen 0,2 µm Filter sterilfiltriert. Die Lösung wird unter aseptischen Bedingungen in Glasvials eingefüllt, mit dem Lyophilisationsstopfen vorverschlossen und in der Gefriertrocknungsanlage getrocknet.

### Referenz beispiel 7: Aminosäuren enthaltende wässerige rViscuminlösung 10 µg/ml (Lyophilisat)

Die Herstellung der Lösungen erfolgt nach der Vorgehensweise wie im Beispiel 4 beschrieben. Entsprechend werden Polysorbat, Tris-Base, Natriumchlorid und die Aminosäur(en) in 80 % der benötigten Menge Wasser für Injektionszwecke gelöst Die Lösungen werden unter aseptischen Bedingungen in Glasampullen oder Glasflaschen eingefüllt. Das Arzneimittel ist bei Lagerungsbedingungen 2 - 8 °C stabil.

| Rezeptur mit Glutaminsäure | |
|---|---|
| rViscumin | 0,01 mg |
| Polysorbat 80 | 1 mg |
| Tris-Base | 2,4 mg |
| HCl (1N) | q.s. pH 8,0 |
| NaCl | 6,5 mg |
| Glutaminsäure | 0,1 mg |
| Wasser für Injektionszwecke | ad 1,0 ml |

| Rezeptur mit Glutaminsäure und Valin | |
|---|---|
| rViscumin | 0,01 mg |
| Polysorbat 80 | 1 mg |
| Tris-Base | 2,4 mg |
| HCl (1N) | q.s. pH 8,0 |
| NaCl | 6,5 mg |
| Glutaminsäure | 0,1 mg |
| Valin | 10 mg |
| Wasser für Injektionszwecke | ad 1,0 ml |

Wird den Lösungen vor der Abfüllung 80 mg Dextran T1 zugesetzt, können ebenfalls Lyophilisate hergestellt werden.

### Referenz beispiel 8: Einfluß verschiedener Aminosäuren auf die Stabilität von carbohydrat-spezifischem rViscumin in gepufferten salinen Lösungen

Im Rahmen der Beschreibung wurde dargestellt, daß Vertreter der Aminosäuren mit sauren, neutralen und basischen Eigenschaften in der Lage sind, das Polypeptid rViscumin in wäßrigen, gepufferten Lösungen zu stabilisieren.

Der in der folgenden Tabelle zusammengefaßt Versuch verdeutlicht den unterschiedlichen Einfluß von Aminosäuren auf die Stabilisierung von rViscumin in gepufferten, wäßrigen, salinen Lösungen bei einem pH-Wert von 8,0.

| Aminosäure | Konzentration mg/ml | Gehalt (%) Ausgangswert | Gehalt (%) 3 Tage Lagerung |
|---|---|---|---|
| keine | - | 100 % | 21,7 % |
| Glutaminsäure | 0,1 | 100 % | 100 % |
| | 10 | 100 % | 100 % |
| Valin | 0,1 | 100 % | 24,2 % |
| | 10 | 100 % | 91,3 % |
| Arginin | 0,1 | 100 % | 74.2 % |
| | 10 | 100 % | 30,5 % |

Wird eine rViscumin-Lösung für drei Tage bei 2 - 8°C gelagert, so kann nach diesem Zeitraum noch 22 % des carbohydrat-spezifischen rViscumins detektiert werden.

Wird der Lösung hingegen die saure Aminosäure Glutaminsäure, die hier als Beispiel für eine saure Aminosäure verwendet wurde, zugesetzt, so kann nach drei Tagen entsprechender Lagerung 100 % des carbohydrat-spezifischen Polypeptids rViscumin wiedergefunden werden. Dieser stabilisierender Effekt wird über den Konzentrationsbereich 0,1-10 mg/ml beobachtet.

Werden neutrale Aminosäuren, wie z.B. Valin, zugesetzt, wird ebenfalls eine Stabilisierung der Polypeptide in wäßriger Lösung beobachtet. Für diese Aminosäure liegt der stabilisierend wirkende Konzentrationsbereich bei 10 mg/ml. Es werden nach drei Tagen Lagerung noch 91 % des Ausgangsgehalts an rViscumin gefunden.

Überraschenderweise konnte auch die Aminosäuren mit basischen Eigenschaften in dem niedrigen Konzentrationsbereich von 0,1 mg/ml ein auf das Protein stabilisierender Effekt beobachtet werden. Die Wiederfindung des Proteins in der entsprechenden Lösung liegt mit einem Gehalt von 74 % deutlich über jenem in dem Kontrollansatz beobachteten Gehalt von 22 %.

Aminosäuren haben demzufolge als Zusätze eine stabilisierend Wirkung sowohl auf wäßrige Lösungen und als auch als Zusätze in getrockneten Zubereitungen (Pulver, Lyophilisate) von rViscumin.

### Referenz beispiel 9: wässerige rViscumin Lösung, Konzentrat zur Infusion 200 µg

Ein Beispiel für die Herstellung einer Lösung, bzw. eines Konzentrates von rViscumin zur Infusion ist im folgenden beschrieben:

| Rezeptur mit Glutaminsäure | |
|---|---|
| rViscumin | 0,20 mg |
| Polysorbat 80 | 10 mg |
| Tris-Base | 24,1 mg |
| HCl (1N) | q.s. pH 8,0 |
| NaCl | 65 mg |
| Glutaminsäure | 1 mg |
| Wasser für Injektionszwecke | ad 10 ml |

Die Herstellung der Lösung erfolgt nach der Vorgehensweise wie im Beispiel 4 beschrieben. Entsprechend werden Polysorbat, Tris-Base, Natriumchlorid und Glutaminsäure werden in 80 % der benötigten Menge Wasser für Injektionszwecke gelöst. Anschließend wird der pH mit HCl (1 N) auf 8,0 eingestellt. In diese Lösung wird das rViscumin eingebracht und gut durchmischt. Mit dem restlichen Wasser wird auf das geforderte Sollvolumen ergänzt und die Lösung über einen 0,2 µm Filter sterilfiltriert. Die Lösung wird unter aseptischen Bedingungen in Glasflaschen eingefüllt. Das Arzneimittel ist bei Lagerungsbedingungen 2 - 8 °C stabil.

Wird der Lösung vor der Abfüllung 800 mg Dextran T1 zugesetzt, kann ebenfalls ein Lyophilisat hergestellt werden.

### Referenz beispiel 10: wässerige rViscumin-Instillationslösung 500 µg

Ein Beispiel für die Herstellung einer Lösung von rViscumin zur Instillation in eine Körperhöhle ist im folgenden beschrieben:

| Rezeptur mit Glutaminsäure | |
|---|---|
| rViscumin | 0,5 mg |
| Polysorbat 80 | 500 mg |
| Tris-Base | 121,1 mg |
| HCl (1N) | q.s. pH 8,0 |
| NaCl | 350 mg |
| Glutaminsäure | 5 mg |
| Wasser für Injektionszwecke | ad 50 ml |

Die Herstellung der Lösung erfolgt nach der Vorgehensweise wie im Beispiel 4 beschrieben. Entsprechend werden Polysorbat, Tris-Base, Natriumchlorid und Glutaminsäure werden in 80 % der benötigten Menge Wasser für Injektionszwecke gelöst. Anschließend wird der pH mit HCl (1 N) auf 8,0 eingestellt. In diese Lösung wird das rViscumin eingebracht und gut durchmischt. Mit dem restlichen Wasser wird auf das geforderte Sollvolumen ergänzt und die Lösung über einen 0,2 µm Filter sterilfiltriert. Die Lösung wird unter aseptischen Bedingungen in Glasflaschen eingefüllt. Das Arzneimittel ist bei Lagerungsbedingungen 2 - 8 °C stabil.

Wird der Lösung vor der Abfüllung 2,0 g Dextran T1 zugesetzt, kann ebenfalls ein Lyophilisat hergestellt werden.

### Referenz beispiel 11: Glucose enthaltende rViscuminlösung 10 µg/ml (Lyophilisat)

Wie oben beschrieben wird in einer bevorzugten Ausführungsform der Erfindung Zucker der rViscumin-Lösung beigesetzt. Ein Beispiel für die Herstellung einer solchen Lösung, die anschließend lyophilisiert wird, ist im folgenden beschrieben:

| Rezeptur mit Glucose und NaCl | |
|---|---|
| rViscumin | 0,01 mg |
| Polysorbat 80 | 1 mg |
| Tris-Base | 24,2 mg |
| HCl (1 N) | q.s. pH 8,0 |
| NaCl | 1 mg |
| Glucose | 80 mg |
| Wasser für Injektionszwecke | ad 1,0 ml |

Polysorbat, Tris-Base und Glucose werden in 80 % der benötigten Menge Wasser für injektionszwecke gelöst. Anschließend wird der pH mit HCl (1N) auf 8,0 eingestellt. In diese Lösung wird das rViscumin eingebracht und gut durchmischt. Mit dem restlichen Wasser wird auf das geforderte Sollvolumen ergänzt. Anschließend wird die Lösung über einen 0,2 µm Filter sterilfiltriert. Die Lösung wird unter aseptischen Bedingungen in Glasvials eingefüllt, mit dem Lyophilisationsstopfen vorverschlossen und in der Gefriertrocknungsanlage getrocknet.

### Referenz beispiel 12: Sorbitol enthaltende rViscuminlösung 10 µg/ml (Lyophilisat)

Wie ebenfalls oben beschrieben wird in anderen bevorzugten Ausführungsform der Erfindung Sorbitol der rViscumin-Lösung beigesetzt. Ein Beispiel für die Herstellung einer solchen Lösung, die anschließend lyophilisiert wir, ist im folgenden beschrieben:

| Rezeptur mit Sorbitol und NaCl | |
|---|---|
| rViscumin | 0,01 mg |
| Polysorbat 80 | 1 mg |
| Tris-Base | 24,2 mg |
| HCl (1 N) | q.s. pH 8,0 |
| NaCl | 1 mg |
| Sorbitol | 80 mg |
| Wasser für Injektionszwecke | ad 1,0 ml |

Polysorbat, Tris-Base und Sorbitol werden in 80 % der benötigten Menge Wasser für Injektionszwecke gelöst. Anschließend wird der pH mit HCl (1 N) auf 8,0 eingestellt. In diese Lösung wird das rViscumin eingebracht und gut durchmischt.
Mit dem restlichen Wasser wird auf das geforderte Sollvolumen ergänzt.
Anschließend wird die Lösung über einen 0,2 µm Filter sterilfiltriert. Die Lösung wird unter aseptischen Bedingungen in Glasvials eingefüllt, mit dem Lyophilisationsstopfen vorverschlossen und in der Gefriertrocknungsanlage getrocknet.

### Referenz beispiel 13: Chitosan enthaltende rViscuminlösung 10 µg/ml (Lyophilisat)

| Rezeptur mit Chitosan und NaCl | |
|---|---|
| rViscumin | 0,01 mg |
| Polysorbat 80 | 1 mg |
| Tris-Base | 24,2 mg |
| HCl (1 N) | q.s. pH 8,0 |
| NaCl | 1 mg |
| Chitosan (nieder molekular) | 80 mg |
| Wasser für Injektionszwecke | ad 1,0 ml |

Polysorbat, Tris-Base und Chitosan werden in 80 % der benötigten Menge Wasser für Injektionszwecke gelöst. Anschließend wird der pH mit HCl (1 N) auf 8,0 eingestellt. In diese Lösung wird das rViscumin eingebracht und gut durchmischt. Mit dem restlichen Wasser wird auf das geforderte Sollvolumen ergänzt. Anschließend wird die Lösung über einen 0,2 µm Filter sterilfiltriert. Die Lösung wird unter aseptischen Bedingungen in Glasvials eingefüllt, mit dem Lyophilisationsstopfen vorverschlossen und in der Gefriertrocknungsanlage getrocknet.

### Beispiel 14: Aerosil enthaltende rViscuminlösung 100 µg/ml (Lyophilisat)

| Rezeptur mit Siliciumdioxid | |
|---|---|
| rViscumin | 0,1 mg |
| Polysorbat 80 | 10 mg |
| Tris-Base | 24,2 mg |
| HCl (1 N) | q.s. pH 8,0 |
| Siliciumdioxid (kolloidal) | 20 mg |
| Dextran T1 | 60 mg |
| Wasser für Injektionszwecke | ad 1,0 ml |

Polysorbat, Tris-Base und Dextran werden in 80 % der benötigten Menge Wasser für Injektionszwecke gelöst. Anschließend wird der pH mit HCl (1 N) auf 8,0 eingestellt. In diese Lösung wird das rViscumin und das kolloidale Siliciumdioxid eingebracht und gut durchmischt. Mit dem restlichen Wasser wird auf das geforderte Sollvolumen ergänzt. Die Lösung wird in Glasvials eingefüllt, mit dem Lyophilisationsstopfen vorverschlossen und in der Gefriertrocknungsanlage getrocknet.

### Referenz beispiel 15: Povidone enthaltende rViscuminlösung 10 µg/ml (Lyophilisat)

| Rezeptur mit Polyvinylpyrrolidon und NaCl | |
|---|---|
| rViscumin | 0,01 mg |
| Polysorbat 80 | 1 mg |
| Tris-Base | 24,2 mg |
| HCl (1 N) | q.s. pH 8,0 |
| NaCl | 1 mg |
| Polyvinylpyrrolidon K 17 | 80 mg |
| Wasser für Injektionszwecke | ad 1,0 ml |

Polysorbat, Tris-Base und Polyvinylpyrrlidon werden in 80 % der benötigten Menge Wasser für Injektionszwecke gelöst. Anschließend wird der pH mit HCl (1 N) auf 8,0 eingestellt. In diese Lösung wird das rViscumin eingebracht und gut durchmischt. Mit dem restlichen Wasser wird auf das geforderte Sollvolumen ergänzt. Anschließend wird die Lösung über einen 0,2 µm Filter sterilfiltriert. Die Lösung wird unter aseptischen Bedingungen in Glasvials eingefüllt, mit dem Lyophilisationsstopfen vorverschlossen und in der Gefriertrocknungsanlage getrocknet.

### Beispiel 16: rViscumin Pulver zur Herstellung einer Lösung, 10 mg rViscumin Lösung zur oralen Einnahme

Beispiele für die Herstellung von rViscumin-Pulver, welche für eine anschließende orale Applikation als Pulver weiterverarbeitet und vor der Anwendung wieder in Wasser gelöst werden, sind im folgenden beschrieben:

| Rezeptur mit Dextran | | |
|---|---|---|
| 1. | rViscumin | 10 mg |
| 2. | Polysorbat 80 | 100 mg |
| 3. | Tris-Base | 24 mg |
| 4. | HCl (1N) | q.s. pH 8,0 |
| 5. | Dextran T1 | 10 g |
| 6. | Saccharose | 10 g |

Die Positionen 1-4 und Teile von 5 (Dextran T1 dient in dieser Rezeptur als lyoprotektive Substanz) werden mit gereinigtem Wasser zu 10 ml gelöst und mittels Gefriertrocknung zu einem Pulver verarbeitet. Dieses Pulver kann gelagert werden.
Das Pulver wird wie in den oben genannten Beispielen mit den weiteren Stoffen gemischt und in 100 ml Flaschen gefüllt. Zur Herstellung der Lösung wird der Feststoff mit Wasser zu 100 ml gelöst.
Die Positionen 1-5 und Teile von 6 (Saccharose dient in dieser Rezeptur als lyoprotektive Substanz) der folgenden Rezeptur werden mit gereinigtem Wasser zu 10 ml gelöst und mittels Gefriertrocknung zu einem Pulver verarbeitet. Dieses Pulver kann gelagert werden. Das Pulver wird wie in den oben genannten Beispielen mit den weiteren Stoffen gemischt und in 100 ml Flaschen gefüllt. Zur Herstellung der Lösung wird der Feststoff mit Wasser zu 100 ml gelöst.

| Rezeptur mit Saccharose (Sucrose) | | |
|---|---|---|
| 1. | rViscumin | 10 mg |
| 2. | Polysorbat 80 | 100 mg |
| 3. | Tris-Base | 120 mg |
| 4. | Glutaminsäure | 10 mg |
| 5. | HCl (1N) | q.s. pH 8,0 |
| 6. | Saccharose | 10 g |
| 7. | Aromen | 0,1 mg |
| 8. | Sorbitol | 10 mg |
| 9. | Wasser | ad 100ml |

### Referenz beispiel 17: rViscumin Pulver zur Herstellung einer Lösung, 10 mg rViscumin Saft zur oralen Einnahme

Ein Beispiel für die Herstellung von rViscumin-Pulver, welche für eine anschließende orale Applikation als Pulver zur Herstellung eines Safts weiterverarbeitet und vor der Anwendung wieder in Wasser gelöst wird, ist im folgenden beschrieben:

| Rezeptur mit Saccharose (Sucrose) | | |
|---|---|---|
| 1. | rViscumin | 10 mg |
| 2. | Polysorbat 80 | 100 mg |
| 3. | Tris-Base | 24 mg |
| 4. | HCl ( 1N) | q.s. pH 8,0 |
| 5. | Saccharose | 25 g |
| 6. | Hydroxyethylcellulose 400 | 700 mg |
| 7. | Xanthan Gummi | 300 mg |
| 8. | Aromen | 0,1 mg |
| 9. | Glycerin 85 % | 1 g |
| 10. | Sorbitol | 10 g |

Die Positionen 1-4 und Teile von 5 werden mit gereinigtem Wasser zu 10 ml gelöst und mittels Gefriertrocknung zu einem Pulver verarbeitet. Dieses Pulver kann gelagert werden. Das Pulver wird in einer bekannten Art mit den weiteren Stoffen gemischt und in 100 ml Flaschen gefüllt. Zur Herstellung des Safts wird der Feststoff mit Wasser zu 100 ml ergänzt und gelöst. Nach Einhalten der Quellzeit ist der Saft zur Einnahme geeignet.

### Beispiel 18: rViscumin Tabletten 0,1 / 0,5 mg

### 250 mg Tablette zur oralen Einnahme

Beispiele für die Herstellung von rViscumin-Tabletten sind im folgenden dargestellt:

| Rezeptur mit Dextran / Cellulose | | | |
|---|---|---|---|
| 1. | rViscumin | 0,1 mg | 0,5 mg |
| 2. | Soja Lecithin | 10 mg | 10 mg |
| 3. | Tris-Base | 24 mg | 24 mg |
| 4. | HCl ( 1N) | q.s. pH 8,0 | q.s. pH 8,0 |
| 5. | Dextran T1 | 100 mg | 100 mg |
| 6. | Cellulose, mikrokristallin | 99 mg | 99 mg |
| 7. | hochdisperses Siliciumdioxid (Aerosil) | 5 mg | 5 mg |
| 8. | quervernetztes Polyvinylpyrrolidon (Kollidon CL) | 5 mg | 5 mg |
| 9. | Magnesiumstearat | 1 mg | 1 mg |

Die Positionen 1-5 werden mit gereinigtem Wasser zu 2 ml gelöst und mittels Gefriertrocknung zu einem Pulver verarbeitet. Dieses Pulver kann gelagert werden. Das Pulver wird in einer bekannten Art mit den weiteren Stoffen zum Pulver gemischt, welches zu Tabletten gepreßt wird. Diese Tabletten können mit einem üblichen Lack versehen werden, der die Freisetzung des Wirkstoffs im Magen verhindert (verzögerte Freisetzung).

| Rezeptur mit Sorbit | | | |
|---|---|---|---|
| 1. | rViscumin | 0,1 mg | 0,5 mg |
| 2. | Polysorbat 80 | 10 mg | 10 mg |
| 3. | Tris-Base | 24 mg | 24 mg |
| 4. | HCl(1N) | q.s. pH 8,0 | q.s. pH 8,0 |
| 5. | Sorbit | 200 mg | 200 mg |
| 6. | hochdisperses Siliciumdioxid (Aerosil) | 5 mg | 5 mg |
| 7. | Natriumcarboxymethylcellulose (Tylopur) | 5 mg | 5 mg |
| 8. | Magnesiumstearat | 1 mg | 1 mg |

Die Positionen 1 - 4 und ein Teil von 5 werden mit gereinigtem Wasser zu 2 ml gelöst und mittels Gefriertrocknung zu einem Pulver verarbeitet. Dieses Pulver kann gelagert werden. Das Pulver wird in einer bekannten Art mit den weiteren Stoffen zum Pulver gemischt, welches zu Tabletten gepreßt wird. Diese Tabletten können mit einem üblichen Lack versehen werden, der die Freisetzung des Wirkstoffs im Magen verhindert (verzögerte Freisetzung).

| Rezeptur mit Dextran | | | |
|---|---|---|---|
| 1. | rViscumin | 0,1 mg | 0,5 mg |
| 2. | Polysorbat 80 | 5 mg | 5 mg |
| 3. | Tris-Base | 12 mg | 12 mg |
| 4. | HCl ( 1N) | q.s. pH 8,0 | q.s. pH 8,0 |
| 5. | Dextran T1 | 40 mg | 40 mg |
| 6. | Cellulose, mikrokristallin | 57 mg | 57 mg |
| 7. | hochdisperses Siliciumdioxid (Aerosil) | 5 mg | 5 mg |

Die Positionen 1-5 werden mit gereinigtem Wasser zu 1 ml gelöst und mittels Gefriertrocknung zu einem Pulver verarbeitet. Dieses Pulver kann gelagert werden. Das Pulver wird in einer bekannten Art mit den weiteren Stoffen zum Pulver gemischt, welches in Hartgelatine Kapseln gefüllt wird.

### Referenz beispiel 19: rViscumin Zäpfchen 1 mg

### 250 Zäpfchen zur Einführung in den Darm

Ein Beispiel für die Herstellung von rViscumin-Zäpfchen ist im folgenden dargestellt:

| Rezeptur mit ß-HP-Cyclodextrin | | |
|---|---|---|
| 1. | rViscumin | 1 mg |
| 2. | Soja Lecithin | 100 mg |
| 3. | Tris-Base | 24 mg |
| 4. | HCl ( 1N) | q.s. pH 8,0 |
| 5. | Dinatrium EDTA | 10 mg |
| 6. | ß-HP-Cyclodextrin | 160 mg |
| 7. | Natriumstearat | 50 mg |
| 8. | Macrogol 300 | 250 mg |
| 9. | Glycerol 85 % | 1,9 g |
| 10. | Gereinigtes Wasser | ad 2,5 g |

Die Positionen 1 - 6 werden mit gereinigtem Wasser zu 2 ml gelöst und mittels Gefriertrocknung zu einem Pulver verarbeitet. Dieses Pulver kann gelagert werden. Das Pulver wird in einer bekannten Art mit den weiteren Stoffen zum Zäpfchen verarbeitet. Die Untermischung des in einem Gemisch von gereinigtem Wasser und Glycerol 85 % gelösten rViscumin Pulvers in die Zäpfchenmatrix erfolgt bei einer kontrollierten Temperatur. Die Masse wird in Formen gepreßt und durch Abkühlen erstarren lassen.

### Referenz beispiel 20: rViscumin Gel 1 mg

### Hydrophiles Gel zur dermalen Anwendung ohne Konservierung

Ein Beispiel für die Herstellung eines hydrophilen rViscumin-Geles zur dermalen Anwendung ist im folgenden dargestellt:

| Rezeptur mit ß-HP-Cyclodextrin | | |
|---|---|---|
| 1. | rViscumin | 1 mg |
| 2. | Poloxamer 166 | 100 mg |
| 3. | Tris-Base | 24 mg |
| 4. | HCl ( 1N) | q.s. pH 8,0 |
| 5. | Dinatrium EDTA | 10 mg |
| 6. | ß-HP-Cyclodextrin | 160 mg |
| 7. | Sorbitanmonostearat (Arlacel 60) | 200 mg |
| 8. | Macrogol-9-stearat | 300 mg |
| 9. | Glycerol 85 % | 500mg |
| 10. | Mittelkettige Triglyceride | 500 mg |
| 11. | Gereinigtes Wasser | ad 10 g |

Die Positionen 1 - 6 werden mit gereinigtem Wasser zu 2 ml gelöst und mittels Gefriertrocknung zu einem Pulver verarbeitet. Dieses Pulver kann gelagert werden. Das Pulver wird in einer bekannten Art mit den weiteren Stoffen zum Gel verarbeitet. Die Untermischung des in gereinigtem Wasser gelösten rViscumin Pulvers in die Gelmatrix erfolgt unterhalb einer Temperatur von 30 °C.

Bei Bedarf kann eine Konservierung mit Natriumbenzoat oder PHB-Estern erfolgen.

### Beispiel 21: rViscumin Pulver zur Inhalation 0,1 / 0,5 mg 1 g Pulver

| Rezeptur mit Dextran / Cellulose | | | |
|---|---|---|---|
| 1. | rViscumin | 0,1 mg | 0,5 mg |
| 2. | Polysorbat 80 | 10 mg | 10 mg |
| 3. | Tris-Base | 24 mg | 24 mg |
| 4. | HCl ( 1N ) | q.s. pH 8,0 | q.s. pH 8,0 |
| 5. | Dextran T1 | 100 mg | 100 mg |
| 6. | Cellulose, mikrokristallin | 860 mg | 860 mg |
| 7. | Natriumcarboxymethylcellulose | 5 mg | 5 mg |

Die Positionen 1-5 werden mit gereinigtem Wasser zu 2 ml gelöst und mittels Gefriertrocknung zu einem Pulver verarbeitet. Dieses Pulver kann gelagert werden. Das Pulver wird in einer bekannten Art mit den weiteren Stoffen zum Pulver gemischt, mikronisiert und mittels Trockenpulver Inhalatoren verabreicht.

### Referenz beispiel 22: Einfluss ausgewählter Kryprotektoren auf die Stabilität von rViscumin

Zubereitungen von rViscumin folgender Zusammensetzung:

| | |
|---|---|
| rViscumin | 10 µg |
| Tris-Base | 12,1 mg |
| Salzsäure 1N zur Einstellung des | pH auf 8.0 |
| Kryoprotektor | 1 /10 mg |
| Natrium EDTA | 10 *µ*g |
| Wasser zur Injektion | ad 1 ml |

werden zu 0,5 ml in Gefriervials gefüllt und im Gefriertrockner mit einer Abkühlrate von 3 K/Stunde auf -35°C abgekühlt, anschließend aufgetaut und die carbohydrat bindende Aktivität des rViscumins in der Lösung entsprechend der Methode wie in Beispiel 4 erläutert bestimmt. Als Kryprotektoren werden Pluronic F 68 und Polysorbat 80 eingesetzt.

Nach dem Auftauen wird eine Wiederfindung der rViscumin Aktivität im Bereich von 98 - 102 % für beide Kryoprotektoren in den beiden Konzentrationen gefunden (Figur 6).

Die zwei Kryoprotektoren Pluronic F68 und Polysorbat 80 sind geeignet im bevorzugten Bereich, wie für die zwei Konzentrationen von 0,1 bis 1.0 % gezeigt, rViscumin während des Einfrierens im Gefriertrocknungsprozess zu stabilisieren.

### Referenz beispiel 23: Einfluss der Proteinkonzentration auf die Stabilität bei Gefriertrocknung

Zubereitungen von rViscumin folgender Zusammensetzung:

| | |
|---|---|
| rViscumin | 10 / 50 / 100 *µ*g |
| Tris-Base | 12,1 mg |
| Salzsäure 1N zur Einstellung des | pH auf 8.0 |
| Polysorbat 80 | 1 mg |
| Natrium EDTA | 10 *µ*g |
| Wasser zur Injektion | ad 1ml |

werden zu 0,5 ml in Gefriervials gefüllt und im Gefriertrockner mit einer Abkühlrate von 3 K/Stunde auf -35°C abgekühlt, anschließend getrocknet.
Trocknungsprogramm:
Primärtrocknung: 8 Stunden bei - 10 °C und 80 kPa Druck gefolgt von Temperaturanstieg auf 10 °C über 8 Stunden und 80 kPa Druck,
Sekundärtrocknung: 6 Stunden bei 30 °C und 10 kPa Druck.

Die gewählten Zubereitungen mit den unterschiedlichen Konzentrationen an rViscumin zeigen unter ausschließlicher Verwendung des Kryoprotektors Polysorbat 80, der zur Stabilisierung von rViscumin während des Gefriervorgangs geeignet ist, eine unzureichende Stabilisierung des Proteins nach Abschluss des Gefriertrocknungsprozesses (Figur 7). Die Stabilität des rViscumin in dem Lyophilisat ist abhängig von der Wahl der Endkonzentration der wässrigen Lösung, so steigt die Wiederfindung der Aktivität von 50 % für die Konzentration 10 µg/ml auf 80 % für die Konzentration 100 µg/ml. Das Beispiel zeigt deutlich, dass in allen rViscumin Konzentrationen der Zusatz geeigneter Lyoprotektoren sich vorteilhaft auf die Stabilität der gefriergetrockneten Arzneiformen auswirken wird.

### Beispiel 24: Einfluss von Mannitol (Mannit) und Mannitol/Dextran auf die Stabilität von rViscumin

Die Zubereitungen von rViscumin (10 µg/ml) folgender Zusammensetzung

| Lösung | Mannit | Mannit/Dextran |
|---|---|---|
| rViscumin | 10 µg | 10 *µ*g |
| Mannit | 20 mg | 20 mg |
| Dextran T1 | | 20 mg |
| TRIS Base | 12,1 mg | 12,1 mg |
| Salzsäure (1N) zur Einstellung des | pH auf 8,0 | |
| Polysorbat 80 | 1 mg | 1 mg |
| Natrium EDTA | 10 *µ*g | 10 *µ*g |
| Wasser zur Injektion | ad 1ml | ad 1ml |

werden zu 0,5 ml in Gefriervials gefüllt und im Gefriertrockner mit einer Abkühlrate von 3 K/Stunde auf -35 °C abgekühlt, anschließend getrocknet.
Trocknungsprogramm:
Primärtrocknung: 8 Stunden bei - 10 °C und 80 kPa Druck gefolgt von Temperaturanstieg auf 10 °C über 8 Stunden und 80 kPa Druck,
Sekundärtrocknung: 6 Stunden bei 30 °C und 10 kPa Druck.

Durch den Zusatz einer suboptimalen Konzentration an Mannit von 2 % wird für rViscumin eine Wiederfindung der Aktivität von 61 % bestimmt (Figur 8). Mannit ist geeignet zur Stabilisierung von rViscumin, da es die Stabilität der gefriergetrockneten rViscumin Lösung 10 µg/ml von 50 % auf 61 % anzuheben vermag. Eine Mischung von Mannit 2% und Dextran T1 2 % hat nach Gefriertrocknung eine Wiederfindung der Aktivität von 74 % zur Folge, woraus geschlossen werden kann, dass auch Dextran allein einen positiven Einfluss auf die Stabilität ausüben kann.

### Beispiel 25: Einfluss von Dextran T1 auf die Stabilität von rViscumin

Die Zubereitungen von rViscumin (10 µg/ml) folgender Zusammensetzung

| | |
|---|---|
| rViscumin | 10 *µ*g |
| Dextran T1 | 0 / 8 / 20 / 40 / 80 mg |
| TRIS Base | 12,1 mg |
| Salzsäure (1N) zur Einstellung des | pH auf 8,0 |
| Polysorbat 80 | 1 mg |
| Natrium EDTA | 10 *µ*g |
| Wasser zur Injektion | ad 1ml |

werden zu 0,5 ml in Gefriervials gefüllt und im Gefriertrockner mit einer Abkühlrate von 3 K/Stunde auf -35 °C abgekühlt, anschließend getrocknet.
Trocknungsprogramm:
Primärtrocknung: 8 Stunden bei - 10 °C und 80 kPa Druck gefolgt von Temperaturanstieg auf 10 °C über 8 Stunden und 80 kPa Druck,
Sekundärtrocknung: 6 Stunden bei 30 °C und 10 kPa Druck.

Für die suboptimale Konzentration von 2 % Dextran T1 wird eine Wiederfindung von 89 % der Aktivität von rViscumin gefunden. Die Stabilität von rViscumin mit Dextran ist deutlich verbessert im Vergleich zu den Resultaten, die unter Verwendung des Gemisch Mannit/Dextran erhalten wurden. Ab einer Dextran Konzentration größer/ gleich 4 % werden im Gefriertocknungsprozess stabile feste Arzneimittelzubereitungen erhalten. Dextran ist geeignet als Lyoprotektor für rViscumin.

### Beispiel 26: Einfluss weiterer Lyoprotektoren

Die Zubereitungen von rViscumin (10 µg/ml) folgender Zusammensetzung

| | |
|---|---|
| rViscumin | 10 *µ*g |
| Lyoprotektor | 80 mg mit Ausnahme von Mannit 20 mg |
| TRIS Base | 12,1 mg |
| Salzsäure (1N) zur Einstellung des | pH auf 8,0 |
| Polysorbat 80 | 1 mg |
| Natrium EDTA | 10 *µ*g |
| Wasser zur Injektion | ad 1ml |

werden zu 0,5 ml in Gefriervials gefüllt und im Gefriertrockner mit einer Abkühlrate von 3 K/Stunde auf -35 °C abgekühlt, anschließend getrocknet.
Trocknungsprogramm:
Primärtrocknung: 8 Stunden bei - 10 °C und 80 kPa Druck gefolgt von Temperaturanstieg auf 10 °C über 8 Stunden und 80 kPa Druck,
Sekundärtrocknung: 6 Stunden bei 30 °C und 10 kPa Druck.

Die Eignung der Zubereitungen mit den Lyoprotektoren in Konzentrationen von 8 % Hydroxyethylstärke 450 (HES 450 8%), von 8 % ß-Hydroxypropyl-Cyclodextrin (β-HP-CD 8%), von 8 % Hydroxyethylstärke 130 (HES 130 8%) und von 8 % Dextran T1 (TRIS 100 Dex T1 8%) und Mannit in einer Konzentration von 2 % (wN) (Man 2%) ist deutlich. Die erst genannten Zubereitungen zeigen nach 8 Stunden bei 60 °C eine Wiederfindung an aktivem rViscumin von größer 60 %, während die Zubereitung mit Mannit unter diesen Bedingungen nur eine reduzierte Stress Stabilität aufweist (Figur 10).

Aus diesen Stress-Stabilitätsdaten können die Bedingungen für die Distribution von Arzneimittel abgeleitet werden. Getrocknete rViscumin Arzneimittel müssen nicht in einer geschlossenen Kühlkette, wie für die wässrigen Zubereitungen erforderlich, transportiert werden.

### Beispiel 27: Vergleichende Lagerungsstabilität von rViscumin Lösung und rViscumin Pulver

Die Zubereitung von rViscumin (10 µg/ml) folgender Zusammensetzung:

| | |
|---|---|
| rViscumin | 10 *µ*g |
| Dextran T10 | 80 mg |
| TRIS Base | 12,1 mg |
| Salzsäure (1N) zur Einstellung des | pH auf 8,0 |
| Polysorbat 80 | 1 mg |
| Natrium EDTA | 10 *µ*g |
| Wasser zur Injektion | ad 1ml |

werden zu 0,5 ml in Gefriervials gefüllt und im Gefriertrockner mit einer Abkühlrate von 3 K/Stunde auf -35°C abgekühlt, anschließend getrocknet.
Trocknungsprogramm:
Primärtrocknung: 8 Stunden bei - 10 °C und 80 kPa Druck gefolgt von Temperaturanstieg auf 10 °C über 8 Stunden und 80 kPa Druck,
Sekundärtrocknung: 6 Stunden bei 30 °C und 10 kPa Druck.
Die Vials werden anschließend unter kontrollierten Bedingungen bei 2 - 8 °C gelagert.

Die Zubereitung von rViscumin (1 µg/ml) folgender Zusammensetzung:

| | |
|---|---|
| rViscumin | 1 *µ*g |
| Natrium monohydrogenphosphat Dihydrat | 17,8 mg |
| Natrium dihydrogenphosphat Dihydrat | 3,13 mg |
| Natriumchlorid | 37,5 mg |
| Polyvidon K 17 | 1 mg |
| Natrium EDTA | 1 mg |
| Wasser zur Injektion | ad 1ml |

wird in Glasampullen gefüllt und unter kontrollierten Bedingungen bei 2 - 8 °C gelagert. Diese Zubereitung ist vergleichbar zu den wässrigen pharmazeutischen Zubereitungen von rViscumin beschrieben in EP 0 751 221 B1.

rViscumin zeigt in dem gefriergetrockneten Pulver nach einer Lagerungsdauer von 52 Wochen eine unveränderte Aktivität. Es ist kein Aktivitätsverlust zu detektieren. Die dem Stand der Technik entsprechende wässrige Zubereitung zeigt eine Stabilität nur über einen kurzen Lagerumgszeitraum und hat nach 6 Wochen Lagerung nur noch 70 % der Aktivität (Figur 11). Es zeigt sich die deutliche Überlegenheit der gefriergetrockneten Zubereitung. Aus diesen Daten kann auf längere Laufzeiten als 1 Jahr für die Pulver förmigen Arzneiformen von rViscumin geschlossen werden, während die wässrige Zubereitung, die nach dem Stand der Technik formuliert wurde, nur eine kurze Laufzeit hat,

Die vorgenannten Beispiele erläutern die beschriebene Erfindung.

### Literatur

Allison SD, Chang BS, Randolph TW, Carpenter JF. Hydrogen Bonding between Sugar and Protein Is Responsible for Inhibition of Dehydration-Induced Protein Unfolding. Arch Biochem Biophys., 1999; 365 (2): 289 - 299.
Allison SD, Manning MC, Randolph TW, Middleton K, Davis A, Carpenter JF. Optimization of Storage Stability of Lyophilized Actin Using Combinations of Disaccharides and Dextran. J Pharm Sci., 2000; 89 (2) 199 - 214.
Bocci V; Mistletoe (viscum album) lectins as cytokine inducers and immunoadjuvant in tumor therapy. J Biol Regul Homeost Agents, 1993; 7(1): 1-6.
Beuth J, Ko HL, Gabius HJ, Pulverer G.; Influence of treatment with the immunomodulatory effective dose of the beta-galactoside-specific lectin from mistletoe on tumor colonization in BALB/c-mice for two experimental model systems. In Vivo, 1991; 5(1): 29-32.
Beuth J, Ko HL, Gabius HJ, Burrichter H, Oette K, Pulverer G.; Behavior of lymphocyte subsets and expression of activation markers in response to immunotherapy with galactoside-specific lectin from mistletoe in breast cancer patients. Clin Investig., 1992; 70(8): 658-61.
Beuth J, Ko HL, Tunggal L, Geisel J, Pulverer G.; [Comparative studies on the immunoactive action of galactoside-specific mistletoe lectin. Pure substance compared to the standardized extract]. Arzneimittelforschung., 1993a;43(2):166-9. German language.
Carpenter JF, Prestrelinski SJ, Arakawa T.;Separation of Freezing- and Drying-Induced Denaturation of Lyophilized Proteins Using Stress-Specific Stabilisation: I. Enzyme Activity and Calorimetric Studies. Arch Biochem Biophys., 1993; 2: 456 - 464.
Carpenter JK, Izutsu K; Freezing- and Drying-Induced Perturbations of Protein Structure and Mechanism of Protein Proteection by Stabilizing Additives. in Rey L, May JC (eds); Freeze-Drying/Lyophilization of Pharmaceutical and Biological Products. New York, Basel: Marcel Dekker Inc. 1999: 123 - 160.
Dulat HJ, von Grumbkow C, Baars W, Schroder N, Wonigeit K, Schwinzer R; Downregulation of human alloimmune responses by genetically engineered expression of CD95 ligand on stimulatory and target cells. Eur J Immunol., 2001; 31 (7): 2217-26
Endo Y, Tsurugi K, Lambert JM.; The site of action of six different ribosome-inactivating proteins from plants on eukaryotic ribosomes: the RNA N-glycosidase activity of the proteins. Biochem Biophys Res Commun., 1988; 150(3): 1032-6.
Endo Y, Oka T, Tsurugi K, Franz H.; The mechanism of action of the cytotoxic lectin from Phoradendron californicum: the RNA N-glycosidase activity of the protein. FEBS Lett., 1989; 248(1-2): 115-8.
Franz H, Haustein B, Luther P, Kuropka U, Kindt A.; Isolation and characterization of mistletoe extracts (Viscum album L.). I. Affinity chromatography of mistletoe extracts on immobilized plasma proteins. Acta Biol Med Ger., 1977, 36(1): 113-7.
Gabius HJ, Walzel H, Joshi SS, Kruip J, Kojima S, Gerke V, Kratzin H, Gabius S.; The immunomodulatory beta-galactoside-specific lectin from mistletoe: partial sequence analysis, cell and tissue binding, and impact on intracellular biosignalling of monocytic leukemia cells. Anticancer Res., 1992; 12(3): 669-75.
Gabius HJ, Gabius S, Joshi SS, Koch B, Schroeder M, Manzke WM, Westerhausen M.; From ill-defined extracts to the immunomodulatory lectin: will there be a reason for oncological application of mistletoe? Planta Med., 1994; 60(1): 2-7.
Gabius HJ und Gabius S; Die Misteltherapie auf dem naturwissenschaftlichen Prüfstand. PZ., 1994; 139, 9-16.
Ganguly C and Das S.; Plant lectins as inhibitors of tumour growth and modulators of host immune response. Chemotherapy, 1994; 40(4): 272-8.
Gerhardt, P, Murray, RGE, Wood, WA, Krieg, NR, (1994) "Methods for General and Moleculat Bacteriology", American Society for Microbiology.
Gloger O., Müller B.W.; Influence of freezing on the pH-shift of different buffer systems. Proceedings 3rd World Meeting on Pharmaceutics, Biopharmaceutics and Pharmaceutical Technology 2000, 967-968
Hajto T. Immunomodulatory effects of iscador: a Viscum album preparation. Oncology, 1986; 43 Suppl 1: 51-65.
Hajto T, Hostanska K, Gabius HJ. Modulatory potency of the beta-galactoside-specific lectin from mistletoe extract (Iscador) on the host defense system in vivo in rabbits and patients. Cancer Res., 1989; 49(17): 4803-8.
Hajto T, Hostanska K, Frei K, Rordorf C, Gabius HJ.; Increased secretion of tumor necrosis factors alpha, interleukin 1, and interleukin 6 by human mononuclear cells exposed to beta-galactoside-specific lectin from clinically applied mistletoe extract. Cancer Res., 1990; 50(11): 3322-6.
Hajto, T, Hostanska, K, (2001); EP 0 602 686 B1
Heiny BM, Beuth J.; Mistletoe extract standardized for the galactoside-specific lectin (ML-1) induces beta-endorphin release and immunopotentiation in breast cancer patients. Anticancer Res., 1994; 14(3B): 1339-42.
Lentzen, H, Eck, J, Baur, A, Zinke, H, (1998); EP 0 751 221 B1.
Mannel DN, Becker H, Gundt A, Kist A, Franz H.; Induction of tumor necrosis factor expression by a lectin from Viscum album. Cancer Immunol Immunother., 1991; 33(3): 177-82.
Matzinger P; The JAM test. A simple assay for DNA fragmentation and cell death. J Immunol Methods., 1991; 145(1-2): 185-92.
Old, RW and Primrose, SB; (1992) "Gentechnologie, Eine Einführung" Georg Thieme Verlag Stuttgart New York.
Paques, EP; (1994) EP 0 430 200 B1.
Peumans WJ, Hao Q, Van Damme EJ.; Ribosome-inactivating proteins from plants: more than RNA N-glycosidases? FASEB J., 2001; 15(9): 1493-506
Sambrook et al., (1989) "Molecular Cloning, A Laboratory Manual"; second edition, CSH Press, Cold Spring Harbor.
Woog, H, Gruber, W, Markl, HJ, Demmer, F. (1992), EP 0 306 824 B1.
Woog, H, Gruber, W, Markl, HJ, Winter, G, Demmer, F. (1996), EP 0 607 156 B1.

## Patentansprüche

1. Verfahren zur Herstellung eines Arzneimittels, enthaltend ein Polypeptid, umfassend mindestens ein rekombinantes carbohydratbindendes Polypeptid einer B-Kette eines Ribosomen-inaktivierenden Proteins oder ein funktionelles Fragment davon, wobei
a.) das Polypeptid oder ein funktionelles Fragment dieses Polypeptids fusioniert ist mit einem zytotoxisch wirkenden Peptid zu einem Fusionsprotein;
b.) das Polypeptid oder ein funktionelles Fragment dieses Polypeptids verbunden ist mit einem weiteren Polypeptid, welches eine enzymatische rRNA-N-Glycosidase Aktivität besitzt;
c.) das Polypeptid oder ein funktionelles Fragment dieses Polypeptids verbunden ist mit einem weiteren Polypeptid, bei dem eine enzymatische rRNA-N-Glycosidase Aktivität durch eine andere zytotoxische Aktivität ersetzt wurde, oder
d.) das Polypeptid oder ein funktionelles Fragment dieses Polypeptids verbunden ist mit einem Fusionsprotein; umfassend ein Polypeptid mit einer enzymatischen rRNA-N-Glycosidase Aktivität und/oder einer anderen zytotoxischen Aktivität; in einer langzeit-lagerungsstabilen Form, darüber hinaus gegebenenfalls enthaltend einen pharmakologischen Träger;
umfassend den Schritt des Abkühlens, Einfrierens, Sprühtrockens oder Gefriertrocknens unter Erhalt der pharmakologischen Eigenschaften des Polypeptids in der Lösung, wobei die Lösung **dadurch gekennzeichnet ist, dass** der pH-Wert der Lösung größer als pH 6,0 ist und ein Lösungsmittel enthaltendes Puffersystem die Aufrechterhaltung dieses pH-Wertes gewährleistet, wobei der Lösung für eine Gefriertrocknung mindestens ein Lyoprotektor in einem Endkonzentrationsbereich von 4% bis 10% zugesetzt ist, wobei der Lyoprotektor Dextran(e) ist sind.

2. Verfahren nach Anspruch 1, wobei das weitere Polypeptid, welches mit dem rekombinanten carbohydratbindenden Polypeptid verbunden ist, die A-Kette eines Ribosomen-inaktivierenden Proteins ist.

3. Verfahren nach Anspruch 2, wobei das Ribosomen-inaktivierende Protein ein Ribosomen-inaktivierendes Protein des Typen II ist.

4. Verfahren nach Anspruch 3, wobei das Ribosomen-inaktivierende Protein des Typ II rViscumin ist.

5. Verfahren nach einem der Ansprüche 1 bis 4, wobei der ph-Wert der Lösung zwischen 6,0 und 9,0 liegt.

6. Verfahren nach einem der Ansprüche 1 bis 5, wobei der pH-Wert der Lösung zwischen 7,5 und 8,5 liegt.

7. Verfahren nach einem der Ansprüche 1 bis 6, wobei das oder die Salze des Puffersystems in einem Endkonzentrationsbereich von 5 mM bis 200 mM eingesetzt werden.

8. Verfahren nach einem der Ansprüche 1 bis 7, wobei das oder die Salze des Puffersystems in einem Endkonzentrationsbereich von 100 mM bis 200 mM eingesetzt werden.

9. Verfahren nach einem der Ansprüche 1 bis 8, wobei das oder die Salze des Puffersystems ausgewählt ist/sind aus einer Gruppe umfassend: TRIS/HCl, TRICIN/HCl, HEPES/HCl, Ammoniumcarbonatpuffer, TRIS/Glutaminsäure und TRIS/Asparaginsäure.

10. Verfahren nach einem der Ansprüche 1 bis 9, wobei zur Stabilisierung der pharmakologischen Eigenschaften des Polypeptids die Lösung eine oder mehrere oberflächenaktive Substanzen enthält.

11. Verfahren nach Anspruch 10, wobei die oberflächenaktiven Substanzen nicht-ionische Tenside sind und in einem Endkonzentrationsbereich von 0,01% bis 5,0% eingesetzt werden.

12. Verfahren nach Anspruch 11, wobei die nicht-ionischen Tenside ausgewählt sind aus der Gruppe umfassend: Fettalkohole, Partialglyceride, Polysorbate, Polyoxyethylenfettsäureether und -fettsäureester, Poloxamere (Polyoxypropylen-Polyoxyethylen-Blockpolymere), Saccharidfettsäureester, Polyoxyglycerolfettsäureester und Phosphatide.

13. Verfahren nach Anspruch 12, wobei die Polysorbate ausgewählt sind aus der Gruppe umfassend Polysorbat 80, Polysorbat 20 und Polyoxyethylen-sorbitolether.

14. Verfahren nach Anspruch 12, wobei die Polyoxyethylenfettsäureether und -fettsäureester Macrogolether oder Macrogolester sind.

15. Verfahren nach Anspruch 12, wobei das Poloxamer Pluronic F68, Poloxamer 166 oder Poloxamer 188 ist.

16. Verfahren nach Anspruch 12, wobei die Phosphatide Lecithine sind.

17. Verfahren nach Anspruch 10, wobei die oberflächenaktiven Substanzen amphotere Tenside sind und in einem Endkonzentrationsbereich von 0,01 % bis 5,0% eingesetzt werden.

18. Verfahren nach einem der Ansprüche 1 bis 17, wobei der Lösung für eine Gefriertrocknung Dextran(e) in einem Endkonzentrationsbereich von 4% bis 10% und Kryoprotektoren in einem Endkonzentrationsbereich von 0,01% bis 1,0% zugesetzt ist.

19. Verfahren nach Anspruch 18, wobei Dextran(e) in einem Endkonzentrationsbereich von 4,0% bis 10% und die Kryoprotektoren in einem Endkonzentrationsbereich von 0,05% bis 0,1 % zugesetzt werden.

20. Verfahren nach Anspruch 18 oder 19, wobei als Kryoprotektoren ionische Substanzen verwendet werden.

21. Verfahren nach Anspruch 20, wobei die ionischen Substanzen ausgewählt sind aus der Gruppe umfassend Natriumchlorid, Natriumsulfat, Kaliumchlorid und Kaliumsulfat.

22. Verfahren nach einem der Ansprüche 18 bis 21, wobei die Lyoprotektoren und Kryoprotektoren bei der Gefriertrocknung amorphe Strukturen bilden.

23. Verfahren nach einem der Ansprüche 1 bis 22, wobei die Stabilisatoren Aminosäuren sind, die in einer Endkonzentration von 0,01 bis 50 mg/ml eingesetzt werden.

24. Verfahren nach Anspruch 23, wobei die Aminosäuren ausgewählt sind aus der Gruppe umfassend saure Aminosäuren wie Glutaminsäure und Asparaginsäure, die basische Aminosäure Arginin und die neutrale Aminosäure Valin.

25. Verfahren nach einem der Ansprüche 1 bis 24, wobei das Polypeptid einer B-Kette eines Ribosomen-inaktivierenden Proteins, umfassend mindestens ein rekombinantes carbohydratbindendes Polypeptid oder funktionelles Fragment dieses Polypeptides, in einer Endkonzentration von 10ng/ml bis 10mg/ml eingesetzt wird.

26. Verfahren nach Anspruch 25, wobei das Polypeptid in einer Endkonzentration von 100 ng/ml bis 1 mg/ml eingesetzt wird.

27. Verfahren nach einem der Ansprüche 1 bis 26, darüber hinaus umfassend die Weiterformulierung oder Rekonstitution des Arzneimittels als wässrige oder nichtwässrige Lösung.

28. Verfahren nach Anspruch 27, wobei das Arzneimittel als Injektions-, Instillations- oder Infusionslösung weiterformuliert wird.

29. Verfahren nach einem der Ansprüche 1 bis 26, darüber hinaus umfassend die Weiterformulierung oder Rekonstitution des Arzneimittels zur gastrointestinalen, oralen, nasalen, pulmonalen, dermalen, transdermalen oder lokalen Anwendung.

30. Verfahren nach einem der Ansprüche 1 bis 27, darüber hinaus umfassend die Weiterformulierung des Arzneimittels zu Saft, Kapseln, Tabletten, Zäpfchen oder Gelen.

31. Verfahren nach einem der Ansprüche 1 bis 26, darüber hinaus umfassend die Weiterformulierung des Arzneimittels zu einem Pulver zur Inhalation, welches in einem Inhalator verabreicht werden kann.

32. Arzneimittel, hergestellt nach einem der Verfahren nach Anspruch 1 bis 31.

## Claims

1. A method for the production of a medicament containing a polypeptide comprising at least one recombinant carbohydrate-binding polypeptide of a B-chain of a ribosome-inactivating protein or a functional fragment thereof, wherein
a.) the polypeptide or a functional fragment of this polypeptide is fused to a cytotoxically effective peptide to form a fusion protein;
b.) the polypeptide or a functional fragment of this polypeptide is linked to another polypeptide which has an enzymatic rRNA-N-glycosidase activity;
c.) the polypeptide or a functional fragment of this polypeptide is linked to another polypeptide in which an enzymatic rRNA-N-glycosidase activity has been replaced by another cytotoxic activity; or
d.) the polypeptide or a functional fragment of this polypeptide is linked to a fusion protein, comprising a polypeptide with an enzymatic rRNA-N-glycosidase activity and/or another cytotoxic activity; in a long-term stable form for storage, and beyond that optionally contains a pharmaceutically acceptable carrier;
comprising the step of cooling, freezing, spray drying or lyophilising while retaining the pharmacological properties of the polypeptide in the solution, wherein the solution is **characterized in that**, the pH value of the solution is higher than pH 6.0 and a solvent containing a buffer system which maintains this pH-value, wherein at least a lyoprotector in a final concentration of between 4.0 and 10% is added to the solution for a lyophilization, wherein said lyoprotector is/are a dextran(e).

2. A method of claim 1, wherein the further polypeptide which is linked to the recombinant carbohydrate-binding polypeptide is the A-chain of a ribosome-inactivating protein.

3. A method of claim 2, wherein the ribosome-inactivating protein is a ribosome-inactivating protein of the type II.

4. A method of claim 3, wherein the ribosome inactivating protein is a type II rViscumin.

5. A method according to any one of claims 1 to 4, wherein the pH-value of the solution is between 6.0 and 9.0.

6. A method according to any one of claims 1 to 5, wherein the pH-value of the solution is between 7.5 and 8.5.

7. A method according to any one of claims 1 to 6, wherein the salt(s) of the buffer system is/are used in a end concentration ranging from 5 mM to 200 mM.

8. A method according to any one of claims 1 to 7, wherein the salt(s) of the buffer system is/are used in a end concentration ranging from 100 mM to 200 mM.

9. A method according to any one of claims 1 to 8, wherein the salt(s) of the buffer system comprise one or more salts selected from the group consisting of TRIS/HC1, TRICIN/HC1, HEPES/HC1, ammonium carbonate buffer, TRIS/glutamic acid and TRIS/aspartic acid.

10. A method according to any one of claims 1 to 9, wherein the solution contains one or more surfactants in order to stabilise the pharmacological properties of the polypeptide.

11. A method of claim 10, wherein the surfactants are non-ionic tensides and are used in a final concentration ranging from 0.01 to 5.0%.

12. A method of claim 11, wherein the non-ionic tensides are selected from the group comprising: fatty alcolhols, partial glycerides, polysorbates, polyoxyethylene fatty acid ethers and polyoxyethylene fatty acid esters, poloxamers (polyoxypropylene-polyoxyethylene-block polymers), saccharide fatty acid esters, polyoxyglycerol fatty acid esters and phosphatides.

13. A method of claim 12, wherein the polysorbates are selected from the group consisting of Polysorbate 80, Polysorbate 20 and polyoxyethylene sorbitol ether.

14. A method of claim 12, wherein the polyoxyethylene fatty acid ethers and polyoxyethylene fatty acid esters are macrogol ethers or macrogol esters.

15. A method of claim 12, wherein the poloxamer is Pluronic F68, or poloxamer 188.

16. A method of claim 12, wherein the phosphatides are lecithins.

17. A method of claim 10, wherein the surfactants are amphoteric tensides and are used in a final concentration ranging from 0.01 to 5.0%.

18. A method according to any one of claims 1 to 17, wherein dextran(e) is added in a final concentration ranging from 4.0 to 10% and cryoprotectors are added in a final concentration ranging from 0.01 to 1.0% to the solution for a lyophilization.

19. A method of claim 18, wherein dextran(e) is added in a final concentration ranging from 4.0 to 10% and cryoprotectors are added in a final concentration ranging from 0.05 to 0.1%.

20. A method of claim 19, wherein ionic substances are used as cryoprotectors.

21. A method of claim 20, wherein the ionic substances are selected from the group consisting of sodium chloride, sodium suphate, potassium chloride and potassium sulphate.

22. A method according to any one of claims 18 to 21, wherein the lyoprotectors and cryoprotectors form amorphous structures during lyophilisation.

23. A method according to any one of claims 1 to 22, wherein the solution contains one or more amino acids which are used in a final concentration of from 0.01 to 50 mg/ml.

24. A method of claim 23, wherein the amino acids are selected from the group comprising acidic amino acids such as glutamic acid and aspartic acid, the basic amino acid arginine and the neutral amino acid valine.

25. A method according to any one of claims 1 to 24, wherein the polypeptide of a B-chain of a ribosome-inactivating protein comprising at least a recombinant carbohydrate-binding polypeptide or functional fragment of this polypeptide is used in a final concentration of from 10 ng/ml to 10 mg/ml.

26. A method of claim 25, wherein the polypeptide is used in a final concentration of from 100 ng/ml to 1 mg/ml.

27. A method of claim 1, bexond that comprising the further formulation or reconstitution of the medicament as aqueous or non-aqueous solution.

28. A method of claim 27, wherein the medicament is further formulated as injection solution, instillation solution or infusion solution.

29. A method according to any one of claims 1 to 26, beyond that comprising the further formulation or reconstitution of the medicament for gastrointestinal, oral, nasal, pulmonary, dermal, transdermal or local application.

30. A method according to any one of claims 1 to 27, beyond that comprising the further formulation of the medicament into juice, capsules, tablets, suppositories or gels.

31. A method according to any one of claims 1 to 26, beyond that comprising the further formulation of the medicament into a powder for inhalation which is administered by use of an inhalator.

32. A medicament, produced according to any of the method according to claim 1 to 31.

## Revendications

1. Procédé pour la fabrication d'un médicament, contenant un polypeptide, comprenant au moins un polypeptide recombinant fixant l'hydrate de carbone d'une chaîne B d'une protéine inactivant les ribosomes, ou un fragment fonctionnel de celui-ci, dans lequel
a) le polypeptide ou un fragment fonctionnel de ce polypeptide est fusionné avec un peptide agissant de façon cytotoxique, en une protéine de fusion ;
b) le polypeptide ou un fragment fonctionnel de ce polypeptide est relié à un autre polypeptide possédant une activité rARN-N-glycosidase enzymatique;
c) le polypeptide ou un fragment fonctionnel de ce polypeptide est relié à un autre polypeptide, dans lequel une activité rARN-N-glycosidase enzymatique a été remplacée par une autre activité cytotoxique, ou
d) le polypeptide ou un fragment fonctionnel de ce polypeptide est relié à une protéine de fusion ; comprenant un polypeptide avec une activité rARN-N-glycosidase enzymatique et/ou une autre activité cytotoxique ; sous une forme stable au stockage de longue durée, et comprenant en outre un support pharmacologique ;
comprenant une étape de refroidissement, de congélation, de séchage par pulvérisation ou de séchage par lyophilisation, tout en maintenant les propriétés pharmacologiques du polypeptide dans la solution, dans lequel la solution est **caractérisée en ce que** la valeur de pH de la solution est supérieure à pH 6,0, et un système de tampon contenant un solvant garantit le maintien de cette valeur de pH, dans lequel, pour un séchage par lyophilisation, au moins un lyoprotecteur est ajouté à la solution, dans une plage de concentration finale de 4% à 10%, dans lequel le lyoprotecteur consiste en du/des dextrane(s).

2. Procédé selon la revendication 1, dans lequel l'autre polypeptide, lequel est relié au polypeptide recombinant fixant l'hydrate de carbone, est la chaîne A d'une protéine inactivant les ribosomes.

3. Procédé selon la revendication 2, dans lequel la protéine inactivant les ribosomes est une protéine inactivant les ribosomes du type II.

4. Procédé selon la revendication 3, dans lequel la protéine inactivant les ribosomes est du type II rViscumin.

5. Procédé selon l'une des revendications 1 à 4, dans lequel la valeur de pH de la solution varie entre 6,0 et 9,0.

6. Procédé selon l'une des revendications 1 à 5, dans lequel la valeur de pH de la solution varie entre 7,5 et 8,5.

7. Procédé selon l'une des revendications 1 à 6, dans lequel le ou les sel(s) du système de tampon est/sont utilisé(s) dans une plage de concentration finale de 5 mM à 200 mM.

8. Procédé selon l'une des revendications 1 à 7, dans lequel le ou les sel (s) du système de tampon est/sont utilisé(s) dans une plage de concentration finale de 100 mM à 200 mM.

9. Procédé selon l'une des revendications 1 à 8, dans lequel le ou les sel(s) du système de tampon est/sont sélectionné(s) parmi un groupe comprenant : TRIS/HCI, TRICIN/HCI, HEPES/HCI, un tampon ammoniac-carbonate, un TRIS/acide glutamique et un TRIS/acide aspartique.

10. Procédé selon l'une des revendications 1 à 9, dans lequel, pour stabiliser les propriétés pharmacologiques du polypeptide, la solution contient une ou plusieurs substances tensioactives.

11. Procédé selon la revendication 10, dans lequel les substances tensioactives sont des agents tensioactifs non-ioniques, utilisés dans une plage de concentration finale de 0,01% à 5,0%.

12. Procédé selon la revendication 11, dans lequel les agents tensioactifs sont sélectionnés parmi le groupe comprenant : les alcools gras, les glycérides partiels, les polysorbates, les esters d'acides gras polyoxyéthyléniques et les éthers d'acides gras polyoxyéthyléniques, les poloxamères (copolymères blocs polyoxypropylène-polyoxyéthylène), les esters d'acide gras de saccharide, les esters d'acide gras polyoxyglycéroliques et les phosphatides.

13. Procédé selon la revendication 12, dans lequel les polysorbates sont sélectionnés parmi un groupe comprenant le polysorbate 80, le polysorbate 20 et l'éther de sorbitol de polyoxyéthylène.

14. Procédé selon la revendication 12, dans lequel les éthers d'acide gras polyoxyéthylène et les esters d'acide gras polyoxyéthylène sont des éthers de macrogol et des esters de macrogol.

15. Procédé selon la revendication 12, dans lequel le poloxamère est du pluronic F68, du poloxamère 166 ou du poloxamère 188.

16. Procédé selon la revendication 12, dans lequel les phosphatides sont de la lécithine.

17. Procédé selon la revendication 10, dans lequel les substances tensioactives sont des agents tensioactifs amphotères, et sont utilisées dans une plage de concentration finale de 0,01% à 5,0%.

18. Procédé selon l'une des revendications 1 à 17, dans lequel, pour un séchage par lyophilisation, du/des dextrane(s) est/sont ajouté(s) à la solution, dans une plage de concentration finale de 4% à 10%, ainsi que des cryoprotecteurs, dans une plage de concentration finale de 0,01% à 1,0%.

19. Procédé selon la revendication 18, dans lequel du/des dextrane(s) est/sont ajouté(s) dans une plage de concentration finale de 4,0% à 10%, ainsi que des cryoprotecteurs, dans une plage de concentration finale de 0,05% à 0,1%.

20. Procédé selon la revendication 18 ou 19, dans lequel des substances ioniques sont utilisées en tant que cryoprotecteurs.

21. Procédé selon la revendication 20, dans lequel les substances ioniques sont sélectionnées parmi un groupe comprenant du chlorure de sodium, du sulfate de sodium, du chlorure de kalium et du sulfate de kalium.

22. Procédé selon l'une des revendications 18 à 21, dans lequel les lyoprotecteurs et les cryoprotecteurs forment des structures amorphes pendant le séchage par lyophilisation.

23. Procédé selon l'une des revendications 1 à 22, dans lequel les stabilisateurs sont des acides aminés, utilisés dans une plage de concentration finale de 0,01 à 50 mg/ml.

24. Procédé selon la revendication 23, dans lequel les acides aminés sont sélectionnés parmi un groupe comprenant des acides aminés, tels que l'acide glutamique et l'acide aspartique, l'acide aminé basique appelé arginine et l'acide aminé neutre appelé valine.

25. Procédé selon l'une des revendications 1 à 24, dans lequel le polypeptide d'une chaîne B d'une protéine inactivant les ribosomes, comprenant au moins un polypeptide recombinant fixant l'hydrate de carbone, ou un fragment fonctionnel de ce polypeptide, est utilisé dans une plage de concentration finale de 10 ng/ml à 10 mg/ml.

26. Procédé selon la revendication 25, dans lequel le polypeptide est utilisé dans une plage de concentration finale de 100 ng/ml à 1 mg/ml.

27. Procédé selon l'une des revendications 1 à 26, comprenant en outre la préparation consécutive ou la reconstitution du médicament, en tant que solution aqueuse ou non aqueuse.

28. Procédé selon la revendication 27, dans lequel le médicament est préparé en tant que solution à injecter, à instiller ou à infuser.

29. Procédé selon l'une des revendications 1 à 26, comprenant en outre la préparation consécutive ou la reconstitution du médicament pour un usage gastrointestinal, oral, nasal, pulmonaire, dermique, transdermique ou local.

30. Procédé selon l'une des revendications 1 à 27, comprenant en outre la préparation consécutive du médicament, sous la forme d'un sirop, de capsules, de tablettes, de suppositoires ou de gel.

31. Procédé selon l'une des revendications 1 à 26, comprenant en outre la préparation consécutive du médicament, sous la forme d'une poudre à inhaler, pouvant être administrée dans un inhalateur.

32. Médicament fabriqué conformément au procédé selon les revendications 1 à 31.
